# EUROPÄISCHE PATENTSCHRIFT

(11) **EP 1 092 721 B1**
(45) Veröffentlichungstag und Bekanntmachung des Hinweises auf die Patenterteilung: **19.12.2007**
(21) Anmeldenummer: 00120067.4
(22) Anmeldetag: 15.09.2000
(51) Int. Cl.: C07D 493/04, C07H 3/02, C09K 19/58, C09K 19/34, A61K 31/335

(54) **Chirale Glucoside und deren Verwendung als chirale Dotierstoffe zur Herstellung von cholestrisch-flüssigkristallinen Zusammensetzungen**
Chiral glucosides and their use as chiral dopants for the production of cholesteric liquid crystalline compositions
Glucosides chiraux et leur application en tant que agents de dopage pour la fabrication de compositions de cristaux liquides cholestériques

(30) Priorität: 12.10.1999 DE 19949284
(43) Veröffentlichungstag der Anmeldung: 18.04.2001
(73) Patentinhaber: BASF Aktiengesellschaft, 67056 Ludwigshafen (DE)
(72) Erfinder: Prechtl, Frank, Dr., 60318 Frankfurt (DE); Haremza, Sylke, Dr., 69151 Neckargemünd (DE); Meyer, Frank, Dr., 69115 Heidelberg (DE); Parker, Robert, 68161 Mannheim (DE); Vill, Volkmar, Dr., 20255 Hamburg (DE); Gesekus, Gunnar, 22111 Hamburg (DE)

(56) Entgegenhaltungen:
- DE-A- 4 200 819
- E. SMITS ET AL.: "Cholesteric Carbohydrate Liquid Crystals Incorporating an Intact Glucopyranose Moiety" MOL. CRYST. LIQ. CRYST. SCI. TECHNOL., SECT. A, Bd. 299, 1997, Seiten 427-432, XP000944244
- E. SMITS ET AL.: "Non-Amphiphilic Carbohydrate Liquid Crystals Containing an Intact Monosaccharide Moiety" MOL. CRYST. LIQ. CRYST. SCI. TECHNOL., SECT. A, Bd. 126, Nr. 7, 1993, Seiten 185-199, XP000198442
- W. M. HO ET AL.: "Chiral Liquid Crystalline Compounds from D-(+)-Glucose" TETRAHEDRON, Bd. 51, Nr. 27, 1995, Seiten 7373-7388, XP000941606
- R. MIETHCHEN ET AL.: "Amphiphile Fluorglucose- und Fluorscyllitol-Derivate" CHEM. BER., Bd. 126, Nr. 7, 1993, Seiten 1707-1712, XP000198441
- W. V. DAHLHOFF ET AL.: "Mesogenic 4-O-Alkyl-D-Glucoses via Methyl 4,6-O-Alkylidene -D-glucopyranosides" LIEBIGS ANN. CHEM., Nr. 10, 1993, Seiten 1063-1067, XP000198439
- W. V. DAHLHOFF: "Synthesis of Mesogenic 4- and 6-O-alkyl-D-glucitols" LIEBIGS ANN. CHEM., Nr. 5, 1991, Seiten 463-467, XP000941607
- V. VILL ET AL.: "Molekulares Verdrillungsvermögen von Kohlenhydrat-Derivaten" Z. NATURFORSCH., A: PHYS. SCI., Bd. 43, Nr. 12, 1988, Seiten 1119-1125, XP002042394
- K. KINASHI ET AL.: "Syntheses and Mesomorphic Properties of New Liquid Crystalline Materials Involving Piperazine Skeleton" MOL. CRYST. LIQ. CRYST., Bd. 67, Nr. 14, 1981, Seiten 49-58, XP000944378

## Beschreibung

Die Erfindung betrifft chirale Verbindungen und deren Verwendung als chirale Dotierstoffe für nematische oder cholesterische Flüssigkristalle zur Erzeugung farbig, im UV- oder IR-Bereich reflektierender Schichten oder zur Herstellung von flüssigkristallin, cholesterisch geordneten Pigmenten.

Cholesterische Flüssigkristalle (Cholesteric Liquid Crystal; CLC) reflektieren zirkular polarisierte elektromagnetische Strahlung in einem von der helikalen Struktur des CLC abhängigen Wellenbereich. Die zentrale Wellenlänge des Reflexionsbandes wird durch die Ganghöhe p der helikalen Struktur bestimmt, die Breite des Bandes durch die optische Anisotropie der Mesogene. Die zentrale Wellenlänge des Reflexionsbandes, die im folgenden als Reflexionswellenlänge bezeichnet wird, ist vom Betrachtungswinkel abhängig. Die Drehrichtung des reflektierten Lichts entspricht dem Drehsinn der cholesterischen Helix.

Cholesterische Flüssigkristallmischungen enthalten in der Regel eine oder mehrere optisch aktive Komponenten zur Induktion einer chiralen Struktur. Beispielsweise können cholesterische Flüssigkristallmischungen aus einem nematischen Basismaterial und einem oder mehreren optisch aktiven Dotierstoffen bestehen. Dabei erzeugen diese im Nematen entweder eine rechts- oder linkshändige Verdrillung, die die Drehrichtung des reflektierten zirkularpolarisierten Lichts bestimmt.

Als chirale Dotierstoffe für flüssigkristalline Phasen sind zahlreiche Verbindungen bekannt (z.B. aus DE-A 43 42 280, DE-A 195 41 820 und DE-A 196 11 101 sowie aus GB-A-2 314 839 und WO 98/00428).

Für linkshelikale Materialien kommen häufig Cholesterinverbindungen in Frage, die außer der Chiralität ausreichend mesogene Eigenschaften mitbringen, um eine stabile Mesophase zu erzeugen. Solche Verbindungen sind beispielsweise beschrieben von H. Finkelmann, H. Ringsdorf et al., in Makromol. Chem. 179, 829-832 (1978). Diese Verbindungen haben allerdings den Nachteil einer komplizierten Synthese und eines hohen Herstellungspreises.

Es bestand nun die Aufgabe, neue chirale Verbindungen bereitzustellen, die für die Herstellung von cholesterisch-flüssigkristallinen Zusammensetzungen geeignet sind und die die oben genannten Nachteile nicht aufweisen.

Diese Aufgabe wurde erfindungsgemäß gelöst durch die Verwendung von Verbindungen der allgemeinen Formel I, 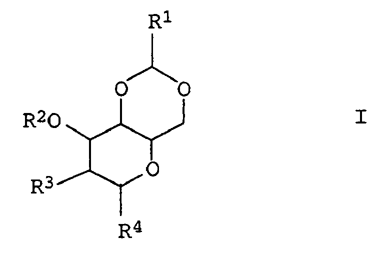 in der die Substituenten unabhängig voneinander folgende Bedeutung haben:
- R¹: Z¹-Y¹-(A¹)ₘ-Y²-M¹-Y³-(A²)ₙ-Y⁴-;
- R²: Wasserstoff, C₁-C₁₂-Alkyl, C₁-C₁₂-Alkylcarbonyl, Aryl, Arylcarbonyl, Z²-Y⁵-(A³)ₒ-Y⁶-M²-Y⁷-(A⁴)ₚ-Y¹¹-;
- R³: Wasserstoff, C₁-C₁₂-Alkyl, Aryl, OR⁵;
- R⁴: Wasserstoff, C₁-C₁₂-Alkyl, Aryl, OR⁶;
- R⁵ und R⁶: Wasserstoff, C₁-C₁₂-Alkyl, C₁-C₁₂-Alkylcarbonyl, Aryl, Arylcarbonyl, Z³-Y⁸-(A⁵)_{q}-Y⁹-M³-Y¹⁰-(A⁶)ᵣ-Y¹²-_{;}
- A¹ bis A⁶: ein Spacer einer Kettenlänge von 1 bis 30 C-Atomen;
- M¹ bis M³: eine mesogene Gruppe;
- Y¹ bis Y¹⁰: eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R)- oder -(R)N-C(**=**O)-, -CH₂-O-, -O-CH₂-, -CH=N-, -N=CH- oder -N=N-;
- Y¹¹ und Y¹²: eine chemische Bindung, -C(=O)-, -O-C(**=**O)-, -CH**=**CH-C(**=**O)-, -(R)N-C(**=**O)-, -CH₂-, -O-CH₂-;
- R: Wasserstoff, C₁-C₄-Alkyl;
- Z¹ bis Z³: Wasserstoff, C₁-C₁₂-Alkyl, eine polymerisierbare Gruppe oder ein Rest, der eine polymerisierbare Gruppe trägt;
- m bis r: 0 oder 1,
wobei die Reste A¹ bis A⁶, Y¹ bis Y¹⁰, Y¹¹ und Y¹² sowie Z¹ bis Z³ gleich oder verschieden sein können, und wobei für den Fall, daß einer oder mehrere der Indices m bis r gleich null sind, mindestens einer der jeweils von A benachbarten Reste Y eine chemische Bindung bedeutet,
als chirale Dotierstoffe für nematische oder cholesterische Flüssigkristalle zur Erzeugung farbig, im UV- oder IR-Bereich reflektierender Schichten oder zur Herstellung von flüssigkristallin, cholesterisch geordneten Pigmenten.

Als Alkylreste für R² bis R⁶ sowie für Z¹ bis Z³ seien verzweigte oder unverzweigte C₁-C₁₂-Alkylketten, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl, n-Undecyl und n-Dodecyl genannt.

Als bevorzugte Alkylreste für R² bis R⁶ seien aus der o.g. Liste die verzweigten oder unverzweigten C₁-C₆-Alkylketten wie z.B. Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl genannt.

Als bevorzugte Alkylreste für Z¹ bis Z³ seien aus der o.g. Liste die verzweigten oder unverzweigten C₄-C₁₀-Alkylketten wie z.B. n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, n-Heptyl, n-Octyl, 2-Ethylhexyl, n-Nonyl und n-Decyl genannt.

Unter Aryl für R² bis R⁶ sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können.

Als Alkyl- bzw. Arylcarbonylreste für R², R⁵ und R⁶ seien Carbonylgruppen mit den o.g. C₁-C₁₂-Alkylketten bzw. den o.g. aromatischen Ringen oder Ringsystemen mit 6 bis 18 Kohlenstoffatomen genannt.

Als Spacer A¹ bis A⁶ kommen alle für diesen Zweck bekannten Gruppen in Betracht. Die Spacer enthalten in der Regel 1 bis 30, vorzugsweise 1 bis 12, besonders bevorzugt 1 bis 6 C-Atome und bestehen aus vorwiegend linearen aliphatischen Gruppen. Sie können in der Kette, z.B. durch nicht benachbarte Sauerstoff- oder Schwefelatome oder Imino- oder Alkyliminogruppen wie beispielsweise Methyliminogruppen, unterbrochen sein. Als Substituenten für die Spacerkette kommen dabei noch Fluor, Chlor, Brom, Cyan, Methyl und Ethyl in Betracht.

Repräsentative Spacer sind beispielsweise:
-(CH₂)ᵤ-, -(CH₂CH₂O)ᵥCH₂CH₂-, -CH₂CH₂SCH₂CH₂-, -CH₂CH₂NHCH₂CH₂-, 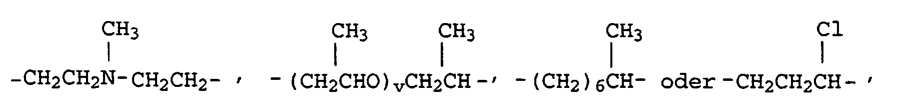 wobei u für 1 bis 12 und v für 1 bis 3 steht.

Bevorzugte Spacer sind Ethylen, Propylen, n-Butylen, n-Pentylen und n-Hexylen.

Ferner ist es auch möglich, einen oder mehrere der mesogenen Reste M¹ bis M³ ohne Spacer direkt mit den zugehörigen Resten Z¹ bis Z³ zu verknüpfen. In diesen Fällen stehen die Indices m, o bzw. q für 0 sowie Y¹/Y², Y⁵/Y⁶ sowie Y⁸/Y⁹ gemeinsam für eine chemische Bindung, insbesondere eine chemische Einfachbindung.

Als Reste M¹ bis M³ können alle bekannten mesogenen Gruppen dienen.

Insbesondere kommen mesogene Gruppen der Formel

(-T-Y¹⁷)_{w}-T-

in Betracht, in der die Variablen folgende Bedeutung haben:
- T: gleiche oder verschiedene zweiwertige, gesättigte oder ungesättigte iso- oder heterocyclische Reste,
- Y¹⁷: Gruppen der Definition für Y¹ bis Y¹⁰,
- w: 0, 1, 2 oder 3,
wobei im Falle w > O, sowohl die Reste T als auch die Gruppen Y¹⁷ jeweils untereinander gleich oder verschieden sein können.

Vorzugsweise ist w gleich 1 oder 2.

Die Reste T können auch durch Fluor, Chlor, Brom, Cyan, Hydroxy oder Nitro substituierte Ringsysteme sein. Bevorzugte Reste T sind:

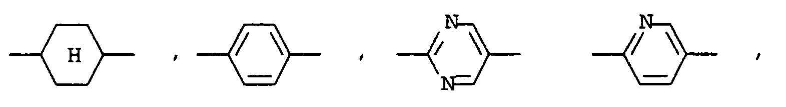

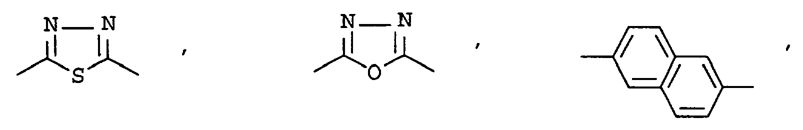

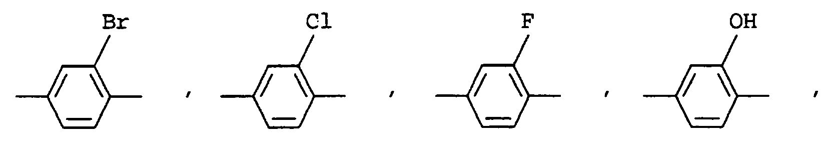

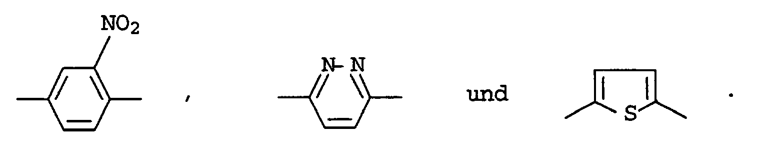

Bevorzugt als mesogene Gruppen M sind z.B.: 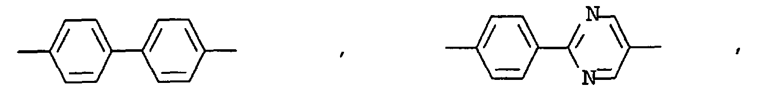 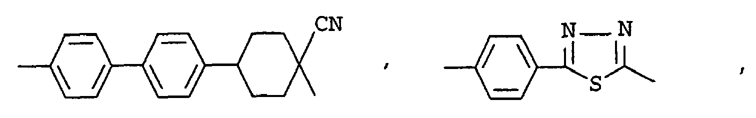 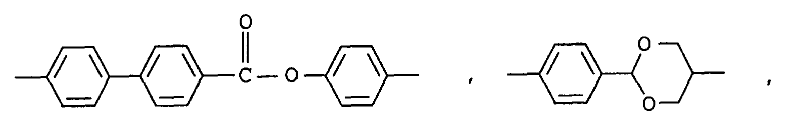 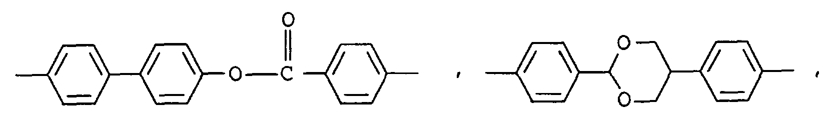 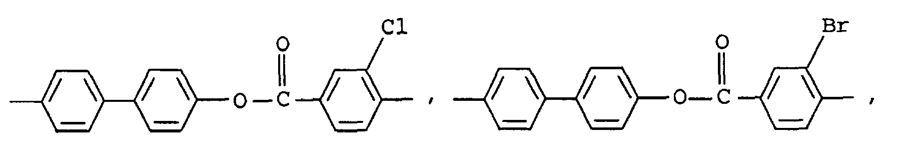 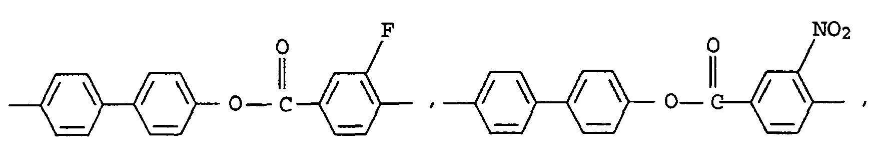 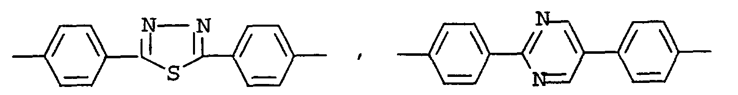 oder

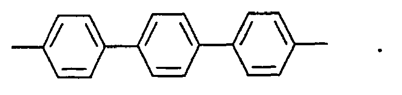

Besonders bevorzugt sind mesogene Gruppen M der folgenden Formeln 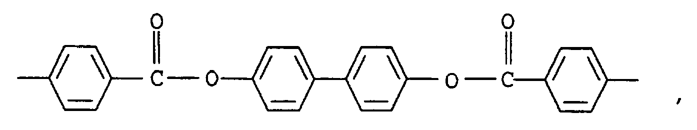 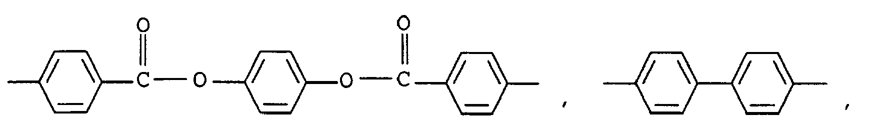 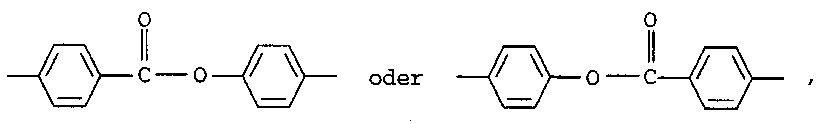 wobei jeder aromatische Ring bis zu drei gleiche oder verschiedene Substituenten aus der folgenden Gruppe tragen kann:
Wasserstoff, C₁-C₂₀-Alkyl, C₁-C₂₀-Alkoxy, C₁-C₂₀-Alkoxycarbonyl, C₁-C₂₀-Monoalkylaminocarbonyl, C₁-C₂₀-Alkylcarbonyl, C₁-C₂₀-Alkylcarbonyloxy, C₁-C₂₀-Alkylcarbonylamino, Formyl, Halogen, Cyan, Hydroxy oder Nitro.

Bevorzugte Substituenten für die aromatischen Ringe sind neben Wasserstoff, Fluor, Chlor, Brom, Cyan, Formyl und Hydroxy vor allem kurzkettige aliphatische Reste wie Methyl, Ethyl, n-Propyl, i-Propyl, n-Butyl, i-Butyl, sek.-Butyl, tert.-Butyl sowie Alkoxy-, Alkoxycarbonyl-, Alkylcarbonyl-, Alkylcarbonyloxy-, Alkylcarbonylamino- und Monoalkylaminocarbonylreste, die diese Alkylgruppen enthalten.

Die äußeren Benzolringe der besonders bevorzugten Gruppen M haben vorzugsweise folgende Substitutionsmuster: 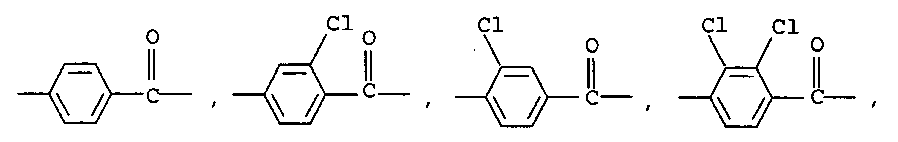 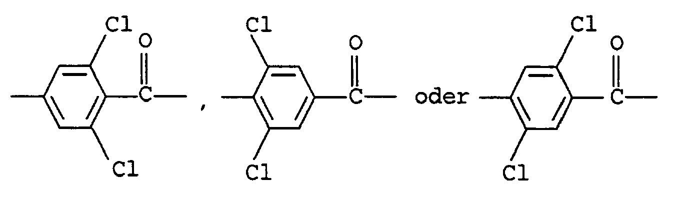 oder sie sind analog mit F, Br, CH₃, OCH₃, CHO, COCH₃, OCOCH₃ oder CN anstelle von Cl substituiert, wobei die Substituenten auch gemischt vorliegen können. Ferner sind die Strukturen 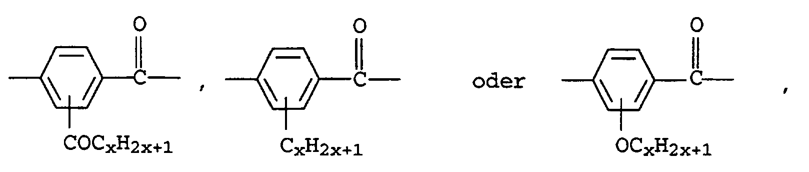 zu nennen, bei denen x 2 bis 20, vorzugsweise 8 bis 15, bedeutet.

Die bevorzugten Substitutionsmuster des mittleren Benzolrings der besonders bevorzugten Gruppen M sind

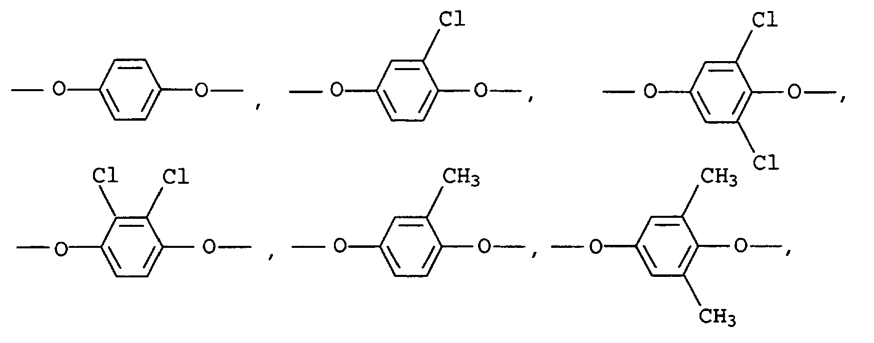

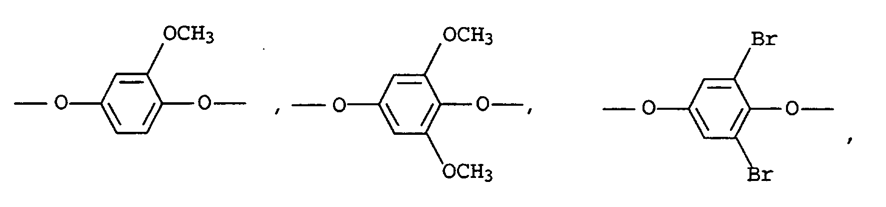

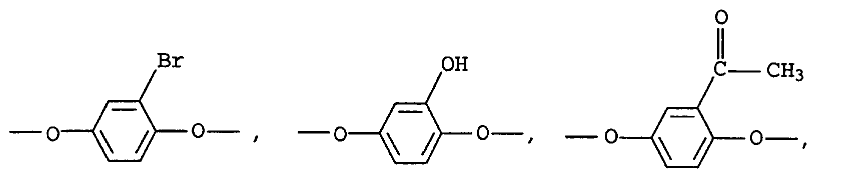

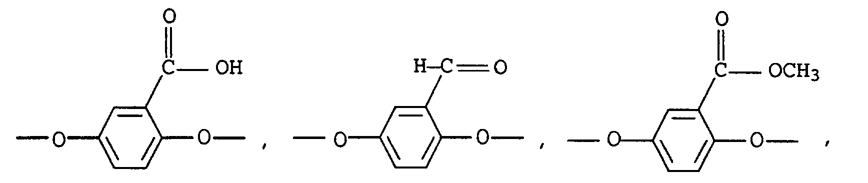

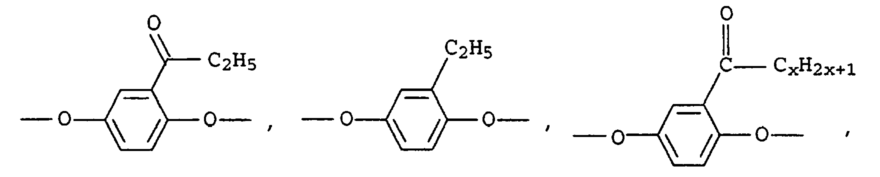

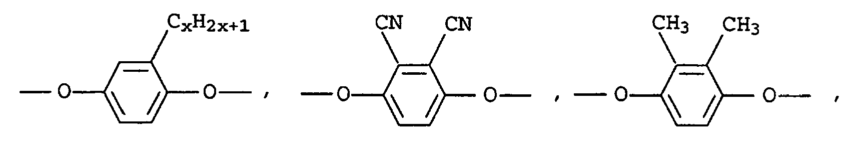

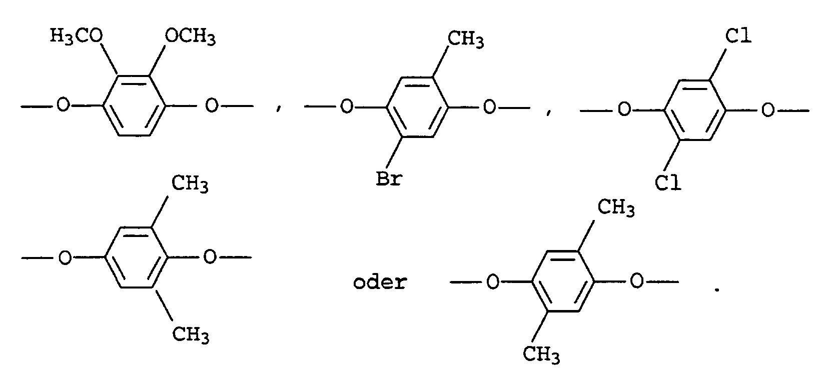

Als Alkylreste seien für R sowie für verzweigte oder unverzweigte C₁-C₄-Alkylketten, bevorzugt Methyl, Ethyl, n-Propyl, 1-Methylethyl, n-Butyl, 1-Methylpropyl-, 2-Methylpropyl oder 1,1-Dimethylethyl genannt.

Bevorzugte Reste für Z¹ bis Z³ sind: 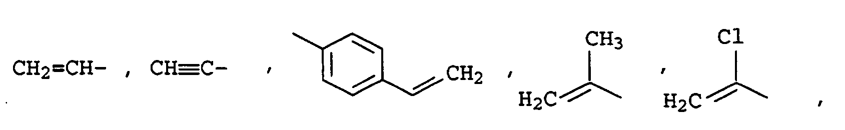 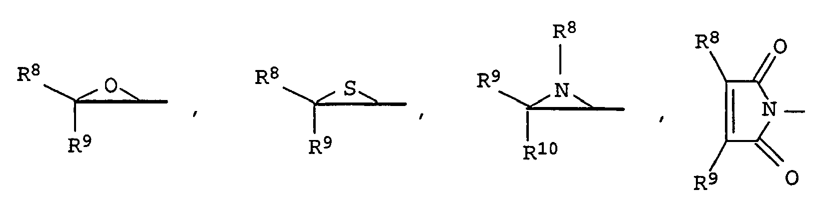 -N=C=O, -N=C=S, -O-C≡N, -COOH, -OH oder NH₂
wobei die Reste R⁸ bis R¹⁰ gleich oder verschieden sein können und Wasserstoff oder C₁-C₄-Alkyl, wie Methyl, Ethyl, n-Propyl, isoPropyl, n-Butyl, iso-Butyl, sec.-Butyl oder tert.-Butyl bedeuten. Von den reaktiven polymerisierbaren Gruppen können die Cyanate spontan zu Cyanuraten trimerisieren und sind daher bevorzugt. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten erfindungsgemäßen Verbindung vorhanden sein, die mit der ersteren gemischt wird, oder sie können durch Hilfsverbindungen, die 2 oder mehr dieser komplementären Gruppen enthalten, in das polymere Netzwerk eingebaut werden.

Als polymerisierbare Gruppierungen sind insbesondere Acrylat und Methacrylat zu nennen.

Besonders bevorzugte Verwendung finden die chiralen Dotierstoffe der allgemeinen Formel Ia, 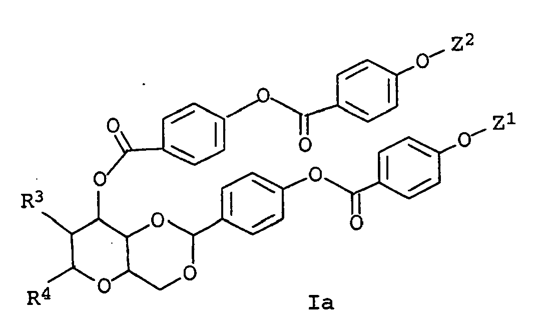 in der die Substituenten unabhängig voneinander folgende Bedeutung haben:
- R³: Wasserstoff;
- R⁴: Wasserstoff;
- Z¹ und Z²: Wasserstoff, C₄-C₁₀-Alkyl, eine polymerisierbare Gruppe oder ein Rest, der eine polymerisierbare Gruppe trägt.
wobei die Definition der Variablen Z¹ und Z² sowohl in der allgemeinen als auch in der bevorzugten Ausführungsform der bereits oben genannten Erläuterung entspricht.

Vorteilhafterweise stellt mindestens einer der Reste Z¹ bis Z³ eine polymerisierbare Gruppe oder einen Rest, der eine polymerisierbare Gruppe enthält, dar.

Die Herstellung der oben genannten Verbindungen erfolgt in an sich bekannter Weise gemäß U. Spohr et al., Can. J. Chem., 1993, 71(11), 1919-1927 sowie den in DE-A-195 32 408, DE-A-44 08 171, EP-A-0 750 029 und in WO 95/16007 beschriebenen Verfahren. Bezüglich näherer Einzelheiten sei auf diese Schriften verwiesen.

Gegenstand der Erfindung sind auch chirale Dotierstoffe der allgemeinen Formel Ib, 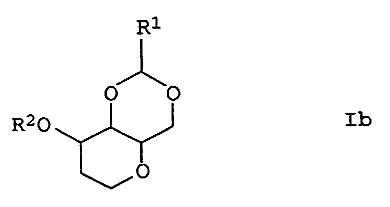 in der die Substituenten unabhängig voneinander folgende Bedeutung haben:
- R¹: Z¹-Y¹-(A¹)ₘ-Y²-M¹-Y³-(A²)ₙ-Y⁴-;
- R²: Wasserstoff, C₁-C₁₂-Alkyl, C₁-C₁₂-Alkylcarbonyl, Aryl, Arylcarbonyl, Z²-Y⁵-(A³)ₒ-Y⁶-M²-Y⁷-(A⁴)ₚ-Y¹¹-;
- A¹ bis A⁴: ein Spacer einer Kettenlänge von 1 bis 30 C-Atomen;
- M¹ und M²: eine mesogene Gruppe;
- Y¹ bis Y⁷: eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R)- oder -(R)N-C(**=**O)-, -CH₂-O-, -O-CH₂-, -CH**=**N-, -N**=**CH- oder -N**=**N-;
- Y¹¹: eine chemische Bindung, -C(=O)-, -O-C(**=**O)-, -CH**=**CH-C(**=**O)-, -(R)N-C(**=**O)-, -CH₂-, -O-CH₂-;
- R: Wasserstoff, C₁-C₄-Alkyl;
- Z¹ und Z²: Wasserstoff, C₁-C₁₂-Alkyl, eine polymerisierbare Gruppe oder ein Rest, der eine polymerisierbare Gruppe trägt;
- m bis p: 0 oder 1,
wobei die Reste A¹ bis A⁴, Y¹ bis Y⁷ sowie Z¹ und Z² gleich oder verschieden sein können, und wobei für den Fall, daß einer oder mehrere der Indices m bis p gleich null sind, mindestens einer der jeweils von A benachbarten Reste Y eine chemische Bindung bedeutet.

Bevorzugt sind solche chiralen Dotierstoffe, in der die Substituenten folgende Bedeutung haben:
- R¹: Z¹-Y¹-(A¹)ₘ-Y²-M¹-Y³-(A²)ₙ-Y⁴-;
- R²: Z²-Y⁵-(A³)ₒ-Y⁶-M²-Y⁷-(A⁴)ₚ-Y¹¹-;
- A¹ und A³: ein Spacer einer Kettenlänge von 1 bis 6 C-Atomen;
- Y¹ bis Y⁷: eine chemische Bindung, -O-, -S-, -C(=O)-, -C(**=**O)-O-, -O-C(**=**O)-, -CH**=**CH-C(**=**O)-O-, -O-C(**=**O)-O-, -C(=O)-N(R)- oder -(R)N-C(**=**O)-, -CH₂-O-, -O-CH₂-, -CH**=**N-, -N**=**CH- oder -N**=**N-;
- Y¹¹: eine chemische Bindung, -C(=O)-, -O-C(**=**O)-, -CH**=**CH-C(**=**O)-, -(R)N-C(**=**O)-, -CH₂-, -O-CH₂-;
- M¹ und M²: ein mesogener Rest aus der Gruppe, bestehend aus: 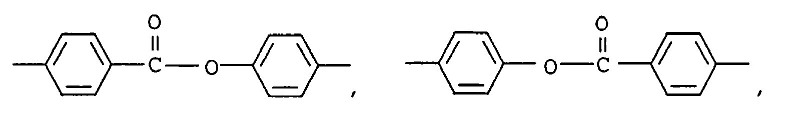 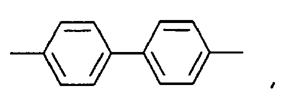 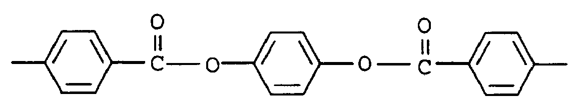 und 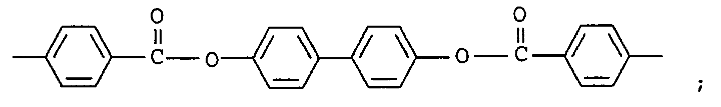
- Z¹ und Z²: Wasserstoff, C₄-C₁₀-Alkyl, eine polymerisierbare Gruppe oder ein Rest, der eine polymerisierbare Gruppe trägt;
- m: 0 oder 1;
- n: 0;
- o: 0 oder 1;
- p: 0,
wobei mindestens einer der Reste Y³ und Y⁴ bzw. Y⁷ und Y¹¹ eine chemische Bindung bedeutet.

Besonders bevorzugt sind chirale Dotierstoffe mit der allgemeinen Formel Ia, 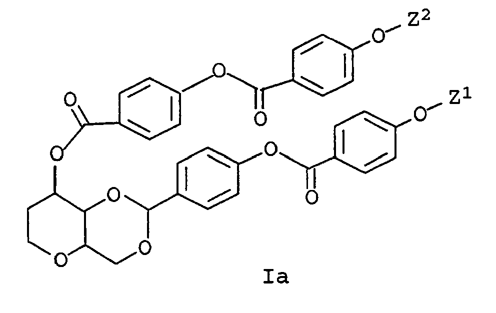 in der die Substituenten unabhängig voneinander folgende Bedeutung haben:
- Z¹ und Z²: Wasserstoff, C₄-C₁₀-Alkyl, eine polymerisierbare Gruppe oder ein Rest, der eine polymerisierbare Gruppe trägt.

Die Definition der o.g. Variablen in der allgemeinen als auch in der bevorzugten Ausführungsform entspricht der bereits oben genannten Erläuterung.

Gegenstand der Erfindung sind außerdem cholesterisch-flüssigkristalline Zusammensetzungen, enthaltend
a) mindestens ein chirales flüssigkristallines Monomeres der allgemeinen Formel I, 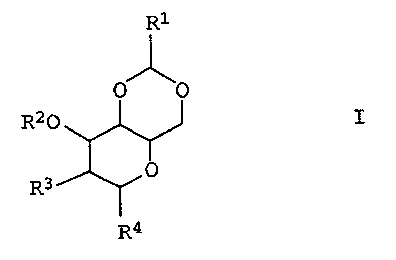 oder
b) eine Mischung aus
b₁) mindestens einem achiralen flüssigkristallinen polymerisierbaren Monomeren der allgemeinen Formel II

   Z⁴-Y¹³-(A⁷)ₛ-Y¹⁴-M⁴-Y¹⁵-(A⁸)ₜ-Y¹⁶-Z⁵ II

   in der die Variablen unabhängig voneinander folgende Bedeutung haben:
   - A⁷ und A⁸: ein Spacer einer Kettenlänge von 1 bis 30 C-Atomen;
   - M⁴: eine mesogene Gruppe;
   - Y¹³ bis Y¹⁶: eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R⁷)- oder -(R⁷)N-C(**=**O)-, -CH₂-O-, -O-CH₂-, -CH**=**N-, -N**=**CH- oder -N=N-;
   - R⁷: Wasserstoff, C₁-C₄-Alkyl;
   - s: 0 oder 1;
   - t: 0 oder 1;
   - Z⁴ und Z⁵: Wasserstoff, C₁-C₁₂-Alkyl, eine polymerisierbare Gruppe oder ein Rest, der eine polymerisierbare Gruppe trägt,
   wobei die Reste A⁷ und A⁸ sowie Y¹³ bis Y¹⁶ gleich oder verschieden sein können, mindestens eine der Variablen Z⁴ oder Z⁵ eine polymerisierbare Gruppe oder einen Rest, der eine polymerisierbare Gruppe trägt, darstellt und für den Fall, daß einer oder beide der Indices s und t gleich null sind, mindestens einer der Reste Y¹³ und Y¹⁴ bzw. Y¹⁵ und Y¹⁶ eine chemische Bindung bedeutet,und
b₂₎ mindestens ein chirales flüssigkristallines Monomer der allgemeinen Formel I.

Im Falle der achiralen flüssigkristallinen Monomeren der Formel II gelten für die polymerisierbaren Gruppen Z⁴ und Z⁵, die Brückenglieder Y¹³ bis Y¹⁶, die Spacer A⁷ und A⁸ sowie für die mesogene Gruppe M⁴ die gleichen Definitionen und bevorzugten Ausführungsformen wie für die entsprechenden Variablen der Formel I.

Ebenso wie in Formel I ist es auch möglich, die mesogene Gruppe direkt mit den Resten Z⁴ oder Z⁵ zu verknüpfen. In diesen Fällen stehen s und/oder t für 0 sowie Y¹³ und Y¹⁴ und/oder Y¹⁵ und Y¹⁶ gemeinsam für eine chemische Bindung.

Das Gemisch b) enthält außerdem noch als chiralen Zusatzstoff b₂) mindestens eine Verbindung der bereits oben beschriebenen Formel I.

Geeignete Dotierstoffe sollten eine hohe Verdrillungsfähigkeit besitzen, so daß geringe Mengen des Dotierstoffs zur Induktion der helikalen Struktur ausreichen. Außerdem sollten die chiralen Dotierstoffe eine gute Kompatibilität zu den flüssigkristallinen Verbindungen zeigen, so daß eine effektive Wechselwirkung zwischen diesen Komponenten ermöglicht wird.

Das Ausmaß der Verdrillung hängt jeweils von der Verdrillungsfähigkeit des chiralen Dotierstoffs und von seiner Konzentration ab. Damit hängt also die Ganghöhe der Helix und wiederum auch die Interferenzwellenlänge von der Konzentration des chiralen Dotierstoffs ab. Es kann daher für den Dotierstoff kein allgemeingültiger Konzentrationsbereich angegeben werden. Der Dotierstoff wird in der Menge zugegeben, mit der die gewünschte UV-Reflektion erzielt wird.

Bevorzugte chirale Zusatzstoffe für b₂) sind Verbindungen der Formel I, in der die Substituenten folgende Bedeutung haben:
- R¹: Z¹-Y¹-(A¹)ₘ-Y²-M¹-Y³-(A²)ₙ-Y⁴-;
- R²: Z²-Y⁵-(A³)ₒ-Y⁶-M²-Y⁷-(A⁴)ₚ-Y¹¹-;
- R³ und R⁴: Wasserstoff;
- A¹ und A³: ein Spacer einer Kettenlänge von 1 bis 6 C-Atomen;
- Y¹ bis Y⁷: eine chemische Bindung, -O-, -S-, -C(=O)-, -C(**=**O)-O-, -O-C(**=**O)-;
- Y¹¹: eine chemische Bindung, -C(=O)-, -O-C(**=**O)-;
- M¹ und M²: ein mesogener Rest aus der Gruppe, bestehend aus: 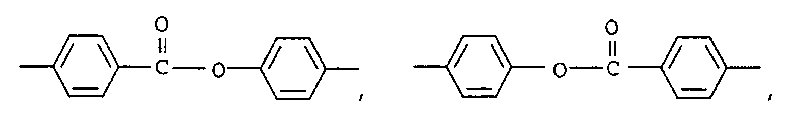 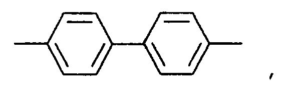 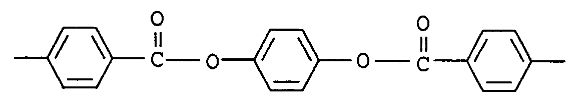 und 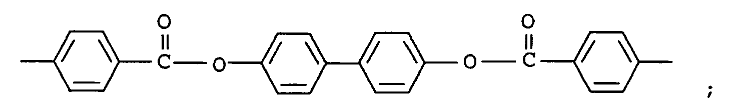
- Z¹: und Z² Wasserstoff, C₄-C₁₀-Alkyl, eine polymerisierbare Gruppe oder ein Rest, der eine polymerisierbare Gruppe trägt;
- m: 0 oder 1;
- n: 0;
- o: 0 oder 1;
- p: 0,
wobei mindestens einer der Reste Y³ und Y⁴ bzw. Y⁷ und Y¹¹ eine chemische Bindung bedeutet.

Als besonders bevorzugte Monomere II sind folgende Strukturen zu nennen: 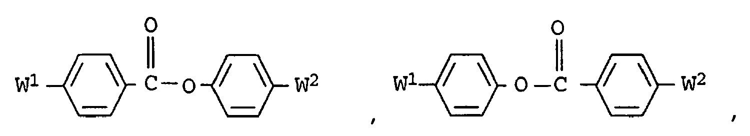 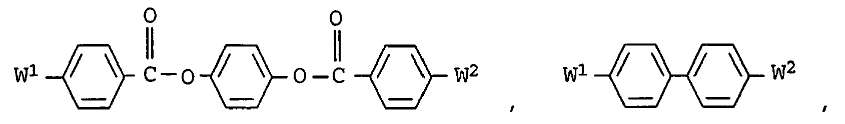 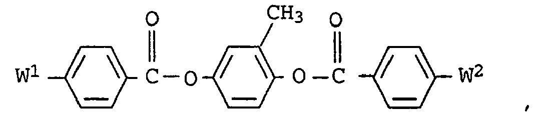 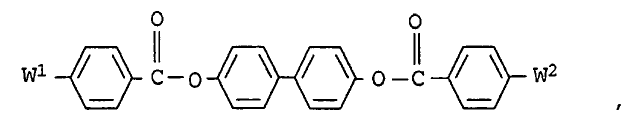 W¹: CH₂=CH-C(=O)-O-(CH₂)₄-O- , W²: -O-(CH₂)₄-O-C(=O)-CH=CH₂ 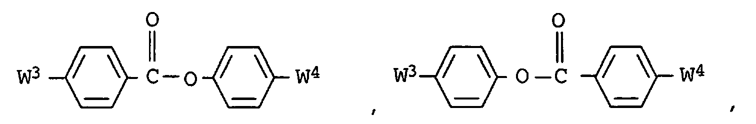 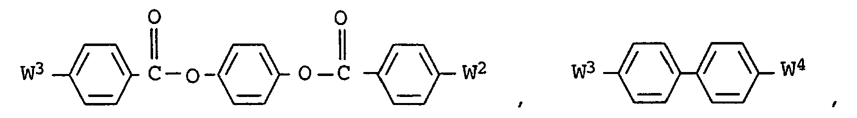 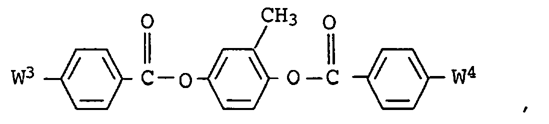 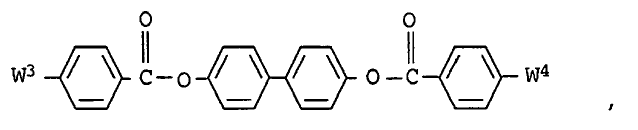 W³: CH₂=CH-C(=O)-O-(CH₂)₆-O- , W⁴: -O-(CH₂)₆-O-C(=O)-CH=CH₂ 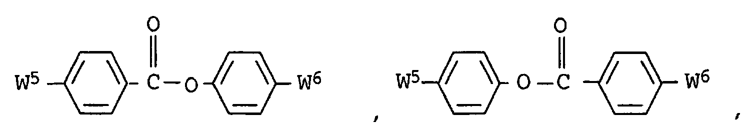 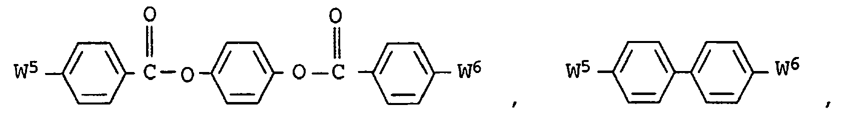 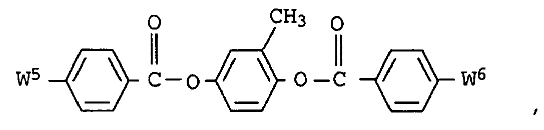 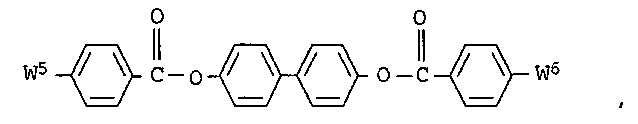 W⁵: CH₂=C(CH₃)-C(=O)-O-(CH₂)₄-O-, W⁶: -O-(CH₂)₄-O-C(=O)-C(CH₃)=CH₂ 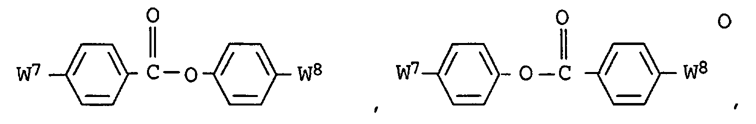 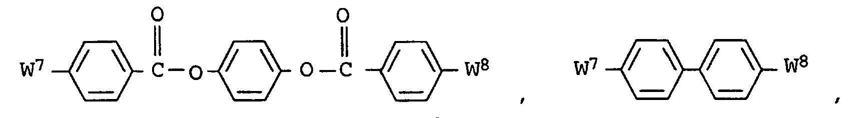 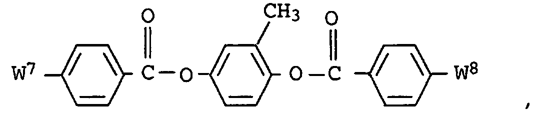 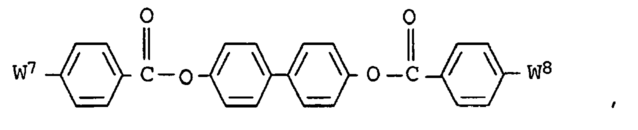 W⁷: CH₂=C(CH₃)-C(=O)-O-(CH₂)₆-O- W⁸: -O-(CH₂)₆-O-C(=O)-C(CH₃)=CH₂ 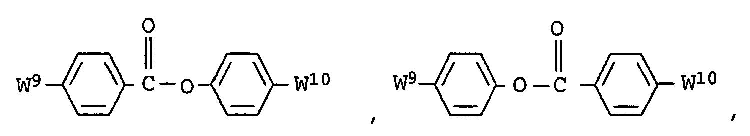 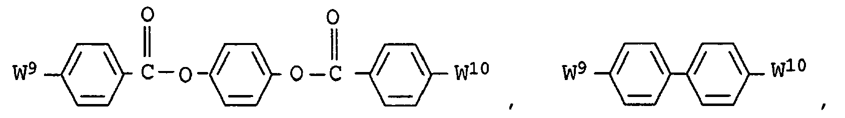 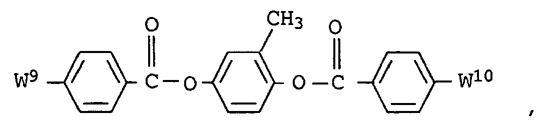 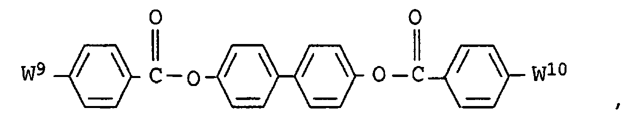 W⁹: CH₂=CH-C(=O)-O-(CH₂)₄-O-C(=O)-O- ,
W¹⁰: -O-(O=)C-O-(CH₂)₄-O-C(=O)-CH=CH₂ 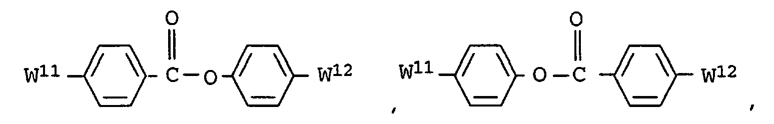 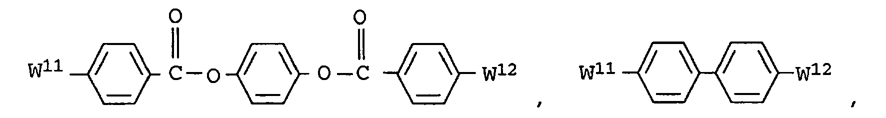 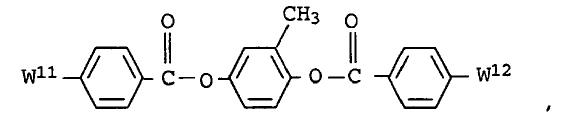 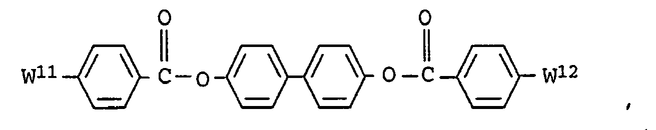 W¹¹: CH₂=CH-C(=O)-O-(CH₂)₆-O-C(=O)-O- ,
W¹²: -O-(O=)C-O-(CH₂)₆-O-C(=O)-CH=CH₂ 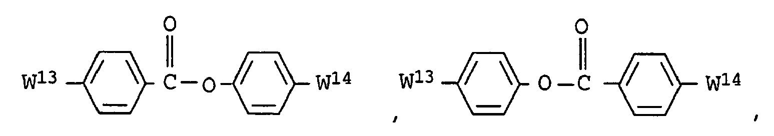 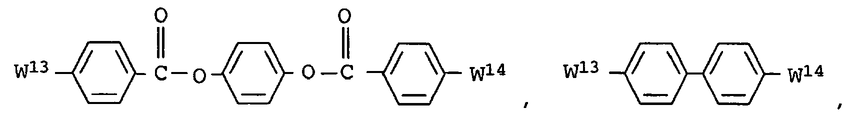 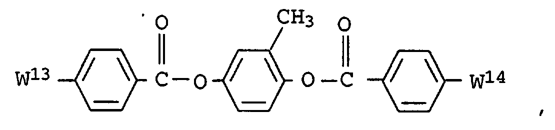 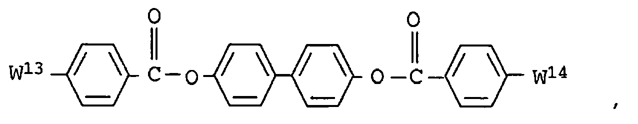 W¹³: CH₂=C(CH₃)-C(=O)-O-(CH₂)₄-O-C(=O)-O- ,
W¹⁴: -O-(O=)C-O-(CH₂)₄-O-C(=O)-C(CH₃)=CH₂ 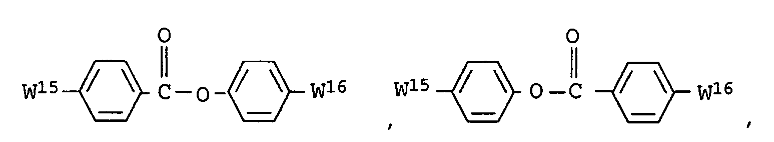 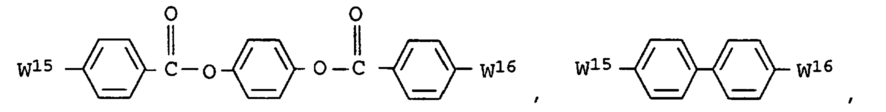 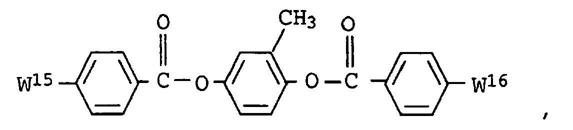 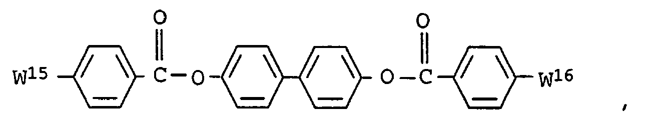 W¹⁵: CH₂=C(CH₃)-C(=O)-O-(CH₂)₆-O-C(=O)-O- ,
W¹⁶_{:} -O-(O=)C-O-(CH₂)₆-O-C(=O)-C(CH₃)=CH₂

Als besonders bevorzugte Monomere I sind folgende Strukturen zu nennen: 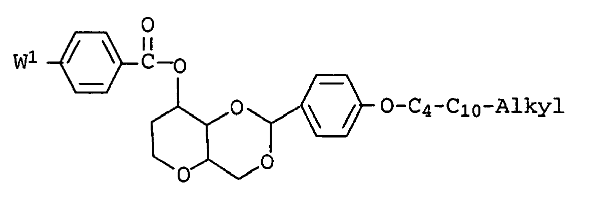 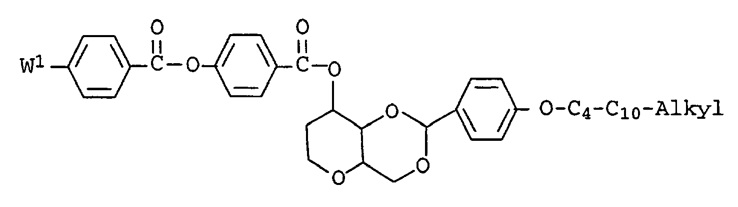 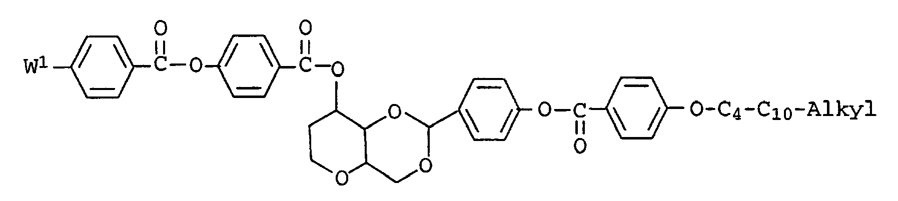 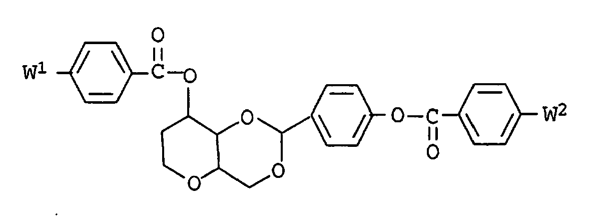 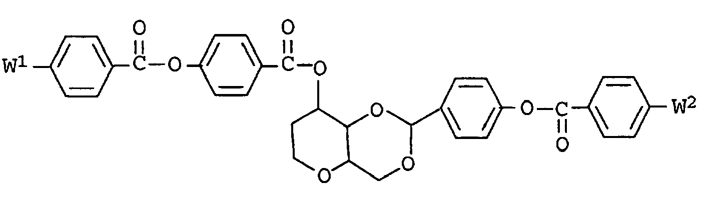 W¹: CH₂=CH-C(=O)-O-(CH₂)₄-O- W²: -O-(CH₂)₄-O-C(=O)-CH=CH₂ 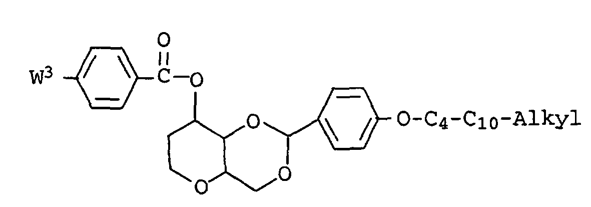 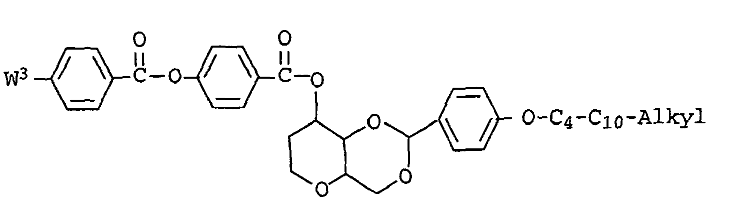 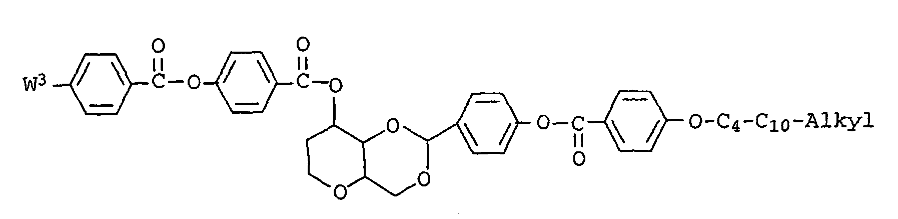 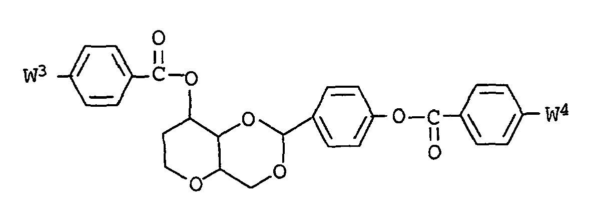 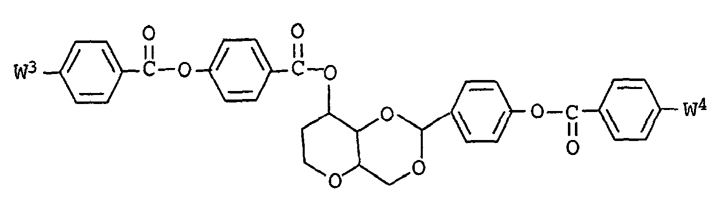 W³: CH₂=CH-C(=O)-O-(CH₂)₆-O- W⁴: -O-(CH₂)₆-O-C(=O)-CH=CH₂ 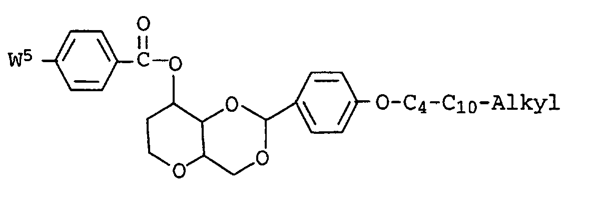 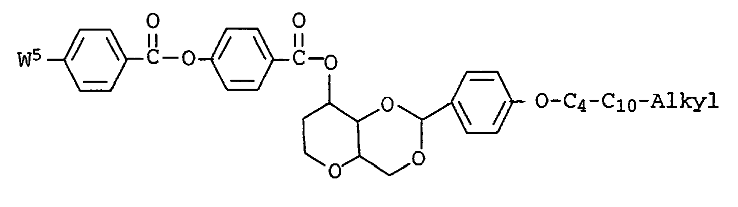 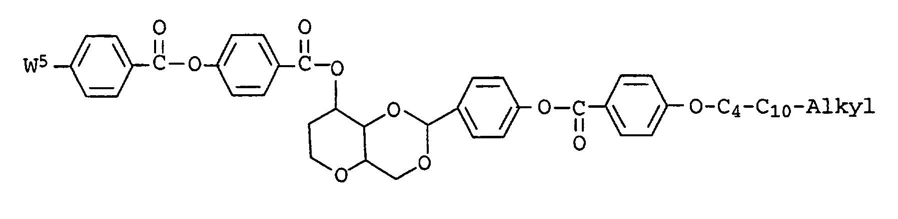 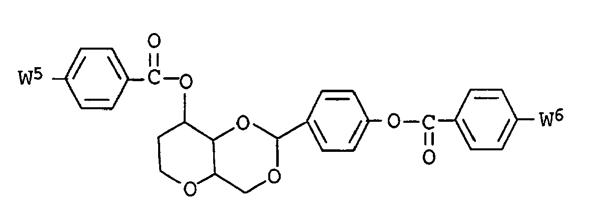 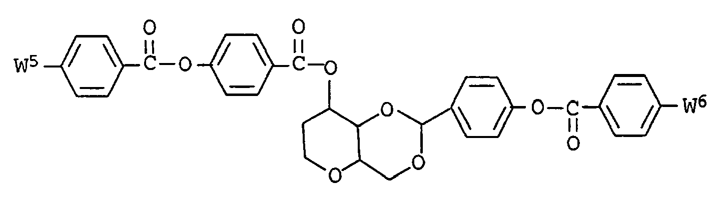 W⁵: CH₂=C(CH₃)-C(=O)-O-(CH₂)₄-O-, W⁶: -O-(CH₂)₄-O-C(=O)-C(CH₃)=CH₂ 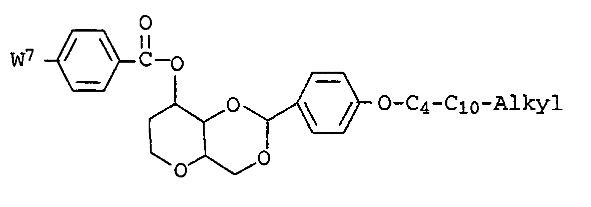 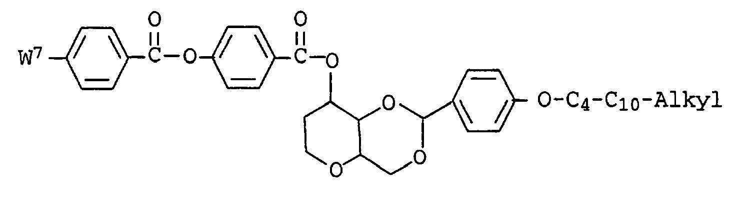 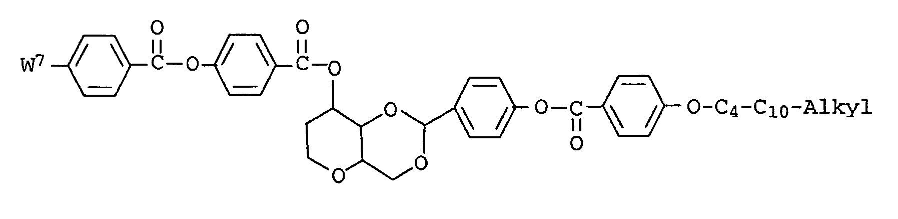 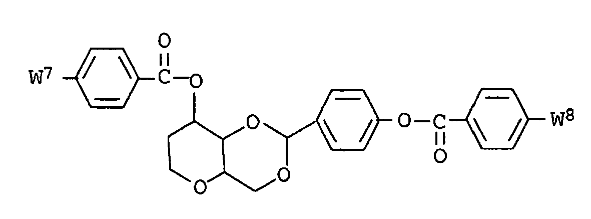 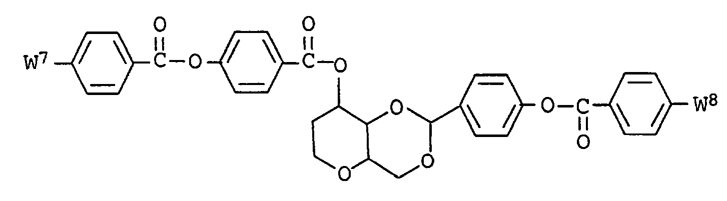 W⁷: CH₂=C(CH₃)-C(=O)-O-(CH₂)₆-O-, W⁸: -O-(CH₂)₆-O-C(=O)-C(CH₃)=CH₂ 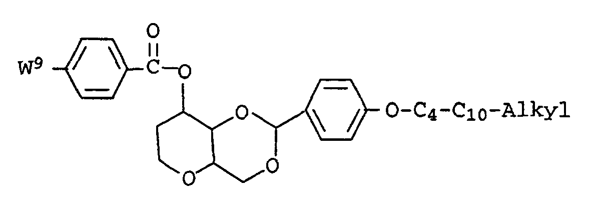 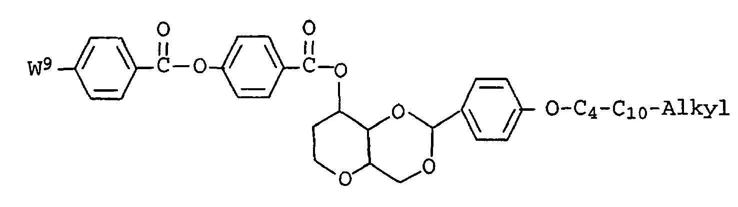 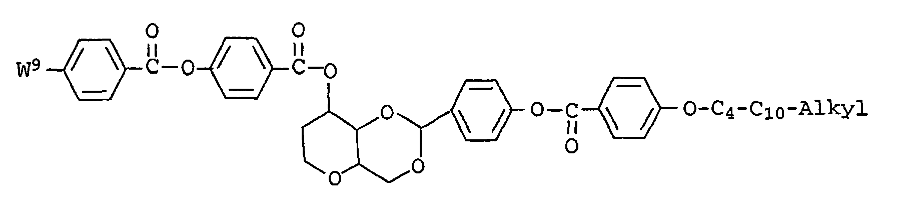 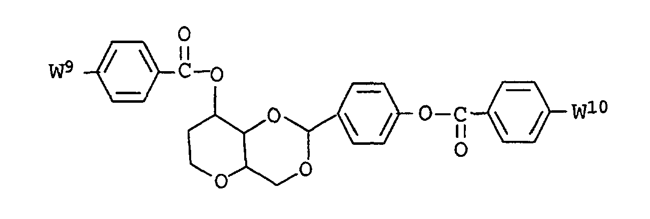 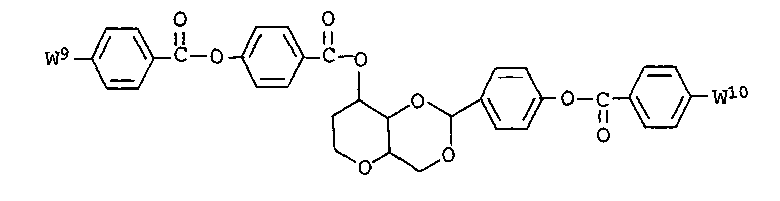 W⁹: CH₂=CH-C(=O)-O-(CH₂)₄-O-C(=O)-O-
W¹⁰: -O-(O=)C-O-(CH₂)₄-O-C(=O)-CH=CH₂ 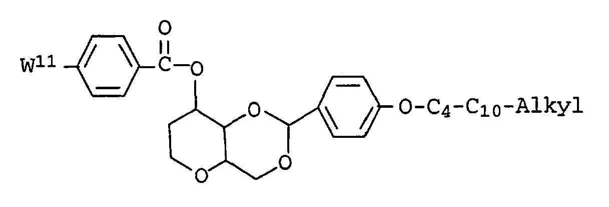 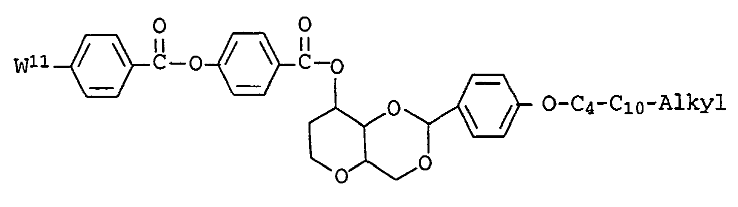 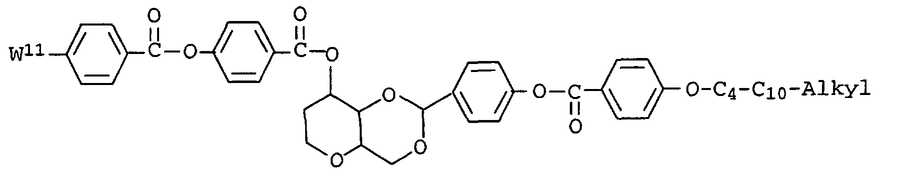 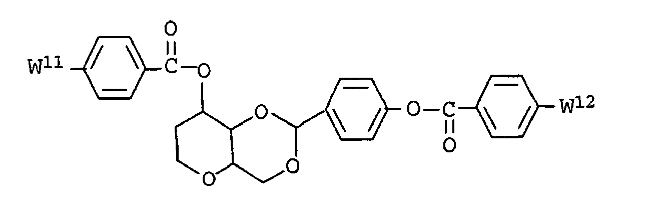 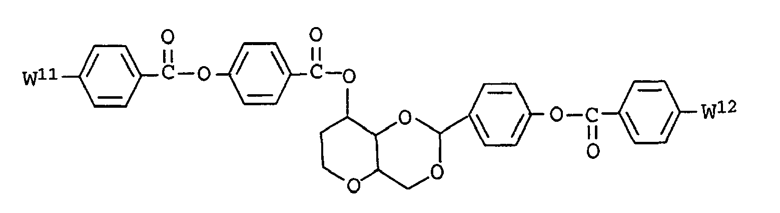 W¹¹: CH₂=CH-C(=O)-O-(CH₂)₆-O-C(=O)-O-
W¹²: -O-(O=)C-O-(CH₂)₆-O-C(=O)-CH=CH₂ 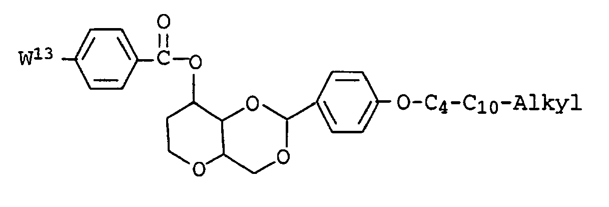 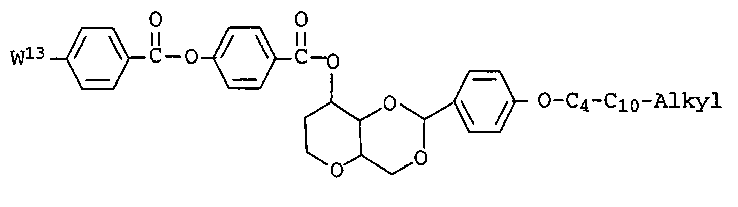 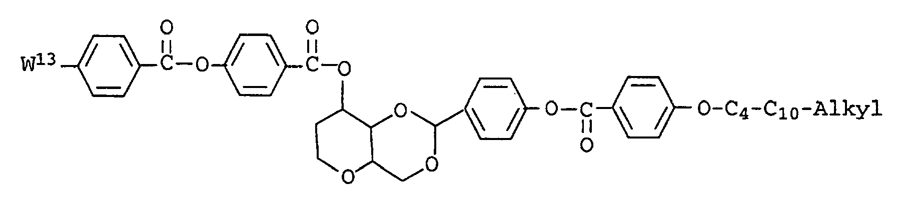 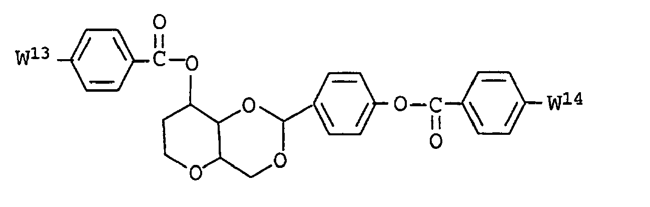 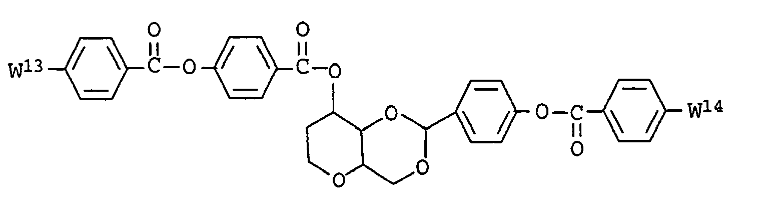 W¹³: CH₂=C(CH₃)-C (=O)-O-(CH₂)₄-O-C(=O)-O-,
W¹⁴: -O-(O=)C-O-(CH₂)₄-O-C(=O)-C(CH₃)=CH₂ 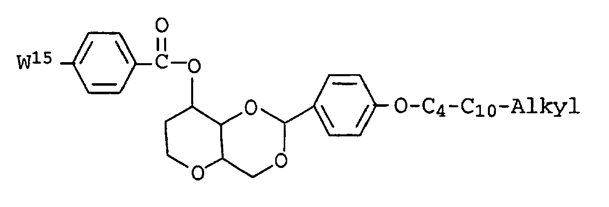 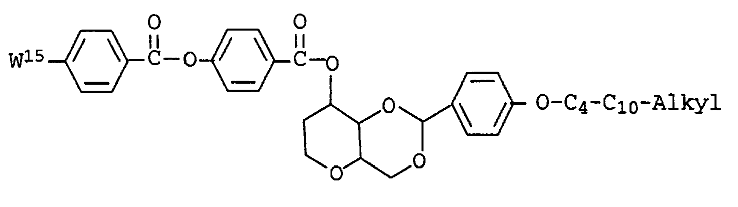 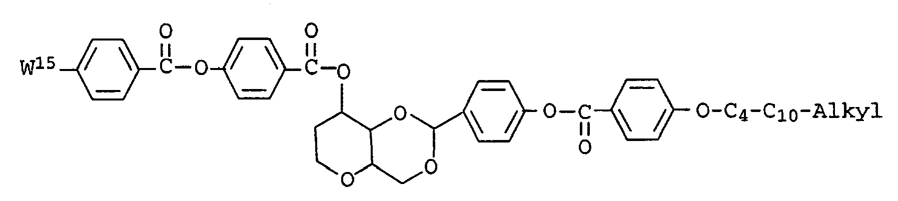 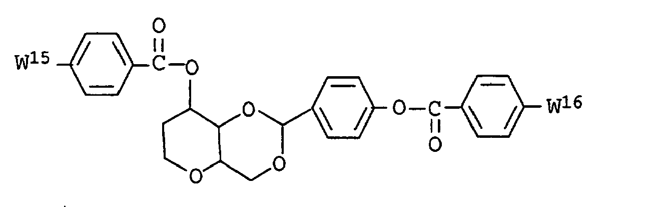 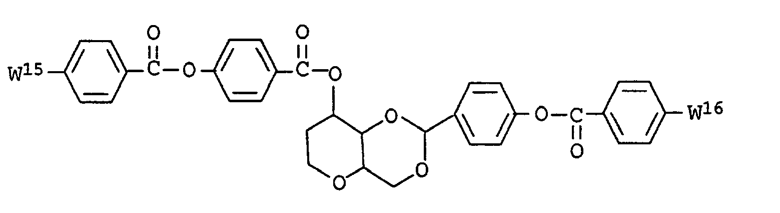 W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂

Die Gewichtsverhältnisse von Komponente II zu Komponente I liegen im Bereich von 99 zu 1 bis 40 zu 60, bevorzugt im Bereich von 99 zu 1 bis 70 zu 30, besonders bevorzugt von 98 zu 2 bis 85 zu 15.

Die obigen cholesterisch-flüssigkristalline Zusammensetzungen sind insbesondere dadurch gekennzeichnet, daß sie linkszirkular polarisiertes Licht reflektieren.

Verwendung der erfindungsgemäßen cholesterisch-flüssigkristallinen Zusammensetzungen in kosmetischen und pharmazeutischen Zubereitungen:

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Neben den bekannten UV-Absorbern, wie z.B. 4-Methoxy-zimtsäure-2-ethylhexylester oder 3-(4'-Methyl)-benzyliden-bornan-2-on werden in kosmetischen und pharmazeutischen Formulierugen häufig auch Lichtschutzmittel eingesetzt, die in Form von Pigmenten die UV-Strahlen reflektieren bzw. absorbieren. Die wichtigsten dieser verwendeten Pigmente sind Titandioxid und Zinkoxid. Bei hohen Einsatzkonzentrationen kann mit Pigmenten eine vollständige Abdeckung der Haut erreicht werden. Dann reflektieren die Partikel allerdings nicht nur UV-Strahlung sondern auch sichtbares Licht, was die häufig nicht gewünschte starke Eigenfärbung pigmenthaltiger Präparate bewirkt.

Während grobteilige Titandioxid Pigmente (Teilchengröße > 500 nm) im UV-B- und UV-A-Bereich vergleichbar wirken, verschiebt sich das Wirkungsspektrum bei feinteiligem Material mit abnehmender Teilchengröße in Richtung UV-B. Dies zeigt, daß die Absorptions-/Reflektionscharakteristik direkt von der Größe und der Verteilung der Teilchen abhängt. Für einen ausgewogenen UV-B- und UV-A-Schutz sind daher bestimmte Teilchengrößenverteilungen erforderlich.

Von Nachteil bei der Verwendung der oben genannten Pigmente erweist sich, daß während der Lagerung der kosmetischen oder pharmazeutischen Lichtschutzmittelformulierungen oftmals Agglomeration, Aggregation und/oder Separation der Pigmentteilchen stattfinden. Die Folge der hierdurch veränderten optischen Eigenschaften kann eine drastisch verringerte Lichtschutzwirkung sein.

Als Alternative zu den o.g. Pigmenten beschreibt DE-A-196 19 460 die Verwendung von Flüssigkristallmischungen mit cholesterischer Phase enthaltend a) flüssigkristalline Organosiloxane, die Dianhydrohexit-Derivate als chirale Gruppen aufweisen und b) chirale monomere Zusatzstoffe, die die gleiche Helizität induzieren wie die jeweiligen flüssigkristallinen Organosiloxane, zur Herstellung von, für kosmetische Zwecke geeignete UV-Schutzschichten in Form von Filmen oder Plättchen. Die hier beschriebenen Flüssigkristallmischungen haben den Nachteil, daß sie sich aufgrund ihrer hohen Viskosität nur unbefriedigend zu Pigmenten verarbeiten lassen.

DE-A-196 29 761 beschreibt kosmetische oder pharmazeutische Zubereitungen, enthaltend Pigmente aus Polyorganosiloxanen mit vom Betrachtungswinkel abhängiger Farbigkeit. Bei den Pigmenten handelt es sich dabei um mindestens eine orientierte vernetzte Substanz einer flüssigkristallinen Struktur mit chiraler Phase. Die hier in den kosmetischen und pharmazeutischen Rezepturen offenbarten Pigmente besitzen zwar gewisse Absorptionseigenschaften im UV-Bereich. Sie haben jedoch für bestimmte Anwendungen den Nachteil, daß es sich hierbei um farbige Verbindungen handelt deren Einsatzgebiet dadurch eingeschränkt ist. Sehr häufig sind aber gerade solche kosmetischen und pharmazeutischen Zubereitungen gefragt, mit denen ein UV-Schutz erzielt wird, bei denen aber eine Färbung der Zubereitung unerwünscht ist.

Gegenstand der Erfindung ist daher auch die Verwendung der o.g. cholesterisch-flüssigkristallinen Zusammensetzungen als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

Die für die Verwendung als UV-Filter in kosmetischen und pharmazeutischen Zubereitungen bevorzugt verwendeten cholesterisch-flüssigkristallinen Zusammensetzungen enthalten eine Mischung aus mindestens einem achiralen flüssigkristallinen polymerisierbaren Monomeren der Formel II und mindestens einem chiralen polymerisierbaren Monomeren der Formel I.

Für die erfindungsgemäße Verwendung der oben genannten cholesterisch-flüssigkristallinen Zusammensetzungen a) und b) als UV-Filter in kosmetischen und pharmazeutischen Zubereitungen können die in diesen Zusammensetzungen enthaltenen Komponenten der Formel I und II direkt in die kosmetischen und pharmazeutischen Zubereitungen eingearbeitet werden.

Bevorzugt werden jedoch die erfindungsgemäß verwendeten cholesterisch-flüssigkristallinen Zusammensetzungen in Form von Pigmenten eingesetzt. Diese Pigmente sind dadurch erhältlich, daß die in den cholesterisch-flüssigkristallinen Zusammensetzungen enthaltenen Monomeren I und II mit Hilfe ihrer polymerisierbaren Gruppen durch radikalische oder ionische Polymerisationsverfahren, welche durch eine photochemische Reaktion gestartet werden können, in hochvernetzte Polymere mit eingefrorener flüssigkristalliner Ordnungsstruktur überführt werden.

Die Herstellung solcher Pigmente ist bekannt und wird u.a. ausführlich in der deutschen Anmeldung P 19738369.6 beschrieben.

Einen Überblick über Verfahren, orientierte Ausgangsstoffe photochemisch zu vernetzen, findet sich darüberhinaus bei C. G. Roffey, Photopolymerisation of Surface Coatings, (1982) John Willey & Sons, Chichester, S. 137 bis 208.

In einer bevorzugten Ausführungsform werden die dreidimensional vernetzbaren polymerisierbaren Monomeren auf eine Unterlage aufgebracht, auf dieser Unterlage vernetzt und nach dem Vernetzen von der Unterlage abgelöst.

Die zu einem Film vernetzten cholesterisch-flüssigkristallinen Zusammensetzungen lassen sich nach der Polymerisation durch Mahlung auf die jeweils erwünschte Korngröße zerkleinern. Je nach der erwünschten Anwendung bzw. je nach Art der kosmetischen oder pharmazeutischen Formulierung können Korngrößen mit einem Durchmesser von 1 bis 1000 µm hergestellt werden. Bevorzugte Korngrößen liegen im Bereich zwischen 1 und 100 µm, besonders bevorzugt zwischen 15 und 50 µm.

Die Dicke der Pigmente liegt zwischen 1 und 100 µm, bevorzugt zwischen 1 und 50 µm besonders bevorzugt zwischen 1,5 und 10 µm.

Die cholesterisch-flüssigkristallinen Zusammensetzungen a) bzw. b), die als Ausgangssubstanzen zur Herstellung der Pigmente geeignet sind, besitzen eine verdrillte Struktur mit einer Ganghöhe, die einer Wellenlänge des Lichtes bis zu 450 nm entspricht. Wie in der bevorzugten Ausführungsform b) gezeigt wird, können sich diese verdrillten Strukturen einer definierten Ganghöhe aus nematischen Strukturen b₁) erhalten werden, indem man ihnen eine chirale Substanz b₂) zusetzt. Art und Anteil der chiralen Substanz bestimmen die Ganghöhe der verdrillten Struktur und damit die Wellenlänge des reflektierten Lichtes. Je nach Chiralität der eingesetzten optisch aktiven Zusatzstoffe kann die Verdrillung der Struktur sowohl links- als auch rechtsgängig sein.

Sogenannte Breitbandreflektoren lassen sich durch einfaches Mischen mehrerer der erfindungsgemäß zu verwendenden cholesterisch flüssigkristallinen Pigmente mit jeweils unterschiedlichen UV-Reflektionsmaxima erzeugen.

Darüber hinaus ist es möglich, durch Mischen von mindestens zwei verschiedenen Pigmenten der cholesterisch-flüssigkristallinen Zusammensetzungen a) und/oder b) mit jeweils entgegengesetzter Verdrillung (Helicität) eine vollständige Reflektion der UV-Strahlen zu erzielen. Pigmente solcher jeweils entgegengesetzt verdrillten, cholesterisch-flüssigkristallinen Strukturen sind beispielsweise durch Zugabe jeweils der einzelnen Spiegelbildisomeren (Enantiomeren) oder Diastereomeren der chiralen Zusatzstoffe b₂) zu dem achiralen flüssigkristallinen polymerisierbaren Monomeren b₁) erhältlich. Die Ganghöhe der jeweils entgegengesetzt verdrillten Strukturen kann dabei gleich oder verschieden sein.

Es ist auch möglich, zunächst die cholesterisch-flüssigkristalline Zusammensetzungen a) oder b) jeweils von entgegengesetzter Verdrillung zu mischen, diese anschließend durch o.g. Vernetzung in die bereits beschriebenen Pigmente zu überführen und als UV-Reflektoren in kosmetischen und pharmazeutischen Formulierungen einzusetzen.

Neben den o.g. Mischungen cholesterisch flüssigkristalliner Pigmente ist es auch möglich Mehrschichtpigmente zu erzeugen, deren einzelne Schichten verschiedene, erfindungsgemäß zu verwendende, dreidimensional vernetzte cholesterisch flüssigkristalline Zusammensetzungen enthalten. Das Design derartiger Mehrschichtpigmente läßt sich vielfältig variieren. So können u.a.
- einzelne Schichten von vernetzten cholesterisch flüssigkristallinen Zusammensetzungen entgegengesetzter Verdrillung oder
- einzelne Schichten von vernetzten cholesterisch flüssigkristallinen Zusammensetzungen gleicher Gangrichtung aber unterschiedlicher Ganghöhe und somit unterschiedlicher Reflektionseigenschaften
übereinander aufgebracht werden.

Bevorzugt sind sogenannte Dreischichtpigmente, bei denen die beiden äußeren Schichten aus jeweils mindestens einer der erfindungsgemäß zu verwendenden vernetzten, cholesterisch flüssigkristallinen Zusammensetzungen besteht und die mittlere Schicht beispielsweise eine Bindemittelmatrix enthalten kann, in dem zusätzlich ein weiterer UV-Absorber eingearbeitet sein kann. Einzelheiten bezüglich Herstellung, Eigenschaften und weiterer Bestandteile solcher mehrschichtigen cholesterischen Pigmente sind der deutschen Patentanmeldung P 19738368.8 zu entnehmen.

Gegenstand der Erfindung sind somit auch die oben beschriebenen Pigmente, insbesondere Mehrschichtpigmente, enthaltend die eingangs genannten cholesterisch-flüssigkristallinen Zusammensetzungen.

Ein Vorteil der erfindungsgemäß verwendeten Pigmente liegt darin, daß deren Zusammensetzung so maßgeschneidert eingestellt werden kann, damit mit diesen Pigmenten die gewünschte UV-Reflektion erzielt werden kann ohne aber eine eigene Farbigkeit (im sichtbaren Bereich) zu zeigen.

Ein weiterer Vorteil der Pigmente liegt in ihren physikalischen Eigenschaften. Aufgrund ihrer geringen Dichte (im Vergleich beispielsweise zu TiO₂) lassen sich die Pigmente gut in Emulsionen einarbeiten, ohne daß es zu Aggregation oder Separation der Pigmentteilchen kommt.

Die erfindungsgemäß zu verwendenden Pigmente lassen sich durch einfaches Abmischen in die kosmetischen und pharmazeutischen Zubereitungen einarbeiten.

Gegenstand der vorliegenden Erfindung sind weiterhin kosmetische und pharmazeutische Zubereitungen, die 0,1 bis 20 Gew.-%, vorzugsweise 0,5 bis 10 Gew.-%, besonders bevorzugt 1 bis 7 Gew.-%, bezogen auf die gesamte Menge der kosmetischen und pharmazeutischen Zubereitung, eine oder mehrere der cholesterisch-flüssigkristallinen Zusammensetzungen aus a) mindestens einem chiralen flüssigkristallinen, polymerisierbaren Monomeren der allgemeinen Formel I mit dem eine cholesterisch-flüssigkristalline Phase mit einer Ganghöhe von kleiner 450 nm erzielt werden kann oder b) einer Mischung aus mindestens einem achiralen flüssigkristallinen polymerisierbaren Monomeren der allgemeinen Formel II und mindestens einem chiralen flüssigkristallinen, polymerisierbaren Monomeren der allgemeinen Formel I mit der eine cholesterisch-flüssigkristalline Phase mit einer Ganghöhe von kleiner 450 nm erzielt werden kann, zusammen mit an sich für kosmetische und pharmazeutische Zubereitungen bekannten, im UV-A- und UV-B-Bereich absorbierenden Verbindungen als Lichtschutzmittel enthalten. Die Variablen der Formeln I und II sowie die Stoffklasse der eingesetzten chiralen Zusatzstoffe entsprechen dabei sowohl in ihrer allgemeinen als auch in ihrer bevorzugten Ausführungsform den bereits oben geschilderten Erläuterungen.

Bevorzugt sind solche der oben genannten kosmetischen und pharmazeutischen Zubereitungen, die die erfindungsgemäß zu verwendenden cholesterisch-flüssigkristallinen Zusammensetzungen in Form der bereits beschriebenen Pigmente, insbesondere in Form von mehrschichtigen Pigmenten enthalten.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurestearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Die erfindungsgemäßen Zubereitungen enthalten vorteilhaft ein oder mehrere Antioxidantien. Als günstige, aber dennoch fakultativ zu verwendende Antioxidantien können alle für kosmetische und/oder dermatologische Anwendungen geeigneten oder gebräuchlichen natürlichen, synthetischen und/oder partialsynthetischen Antioxidantien verwendet werden.

Besonders vorteilhaft werden die Antioxidantien gewählt aus der Gruppe, bestehend aus:

Aminosäuren (z.B. Glycin, Histidin, Tyrosin, Tryptophan) und deren Derivate, Imidazole (z.B. Urocaninsäure) und deren Derivate, Peptide wie D,L,-Carnosin, D-Carnosin, L-Carnosin und deren Derivate (z.B. Anserin), Carotinoide (z.B. β-Carotin, Lycopin) und deren Derivate, Liponsäure und deren Derivate (z.B. Dihydroliponsäure), Aurothioglucose, Propylthiouracil und andere Thioverbindungen (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA, EGTA und deren Derivate, ungesättigte Fettsäuren und deren Derivate (z.B. γ-Linolensäure, Linolsäure, Ölsäure), Folsäure und deren Derivate (z.B. 5-Methyltetrahydrofolsäure), Ubichinon und Ubichinol und deren Derivate, Vitamin C und deren Derivate (z.B. Ascorbylpalmitat, Ascorbylphosphate, Ascorbylacetate), Tocopherole und Derivate (z.B. Tocopherylacetat, Tocotrienol), Vitamin A und Derivate (z.B. Vitamin A-Palmitat), Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Stilbene und deren Derivate.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Zubereitung - betragen. Die Herstellung der Zubereitung kann in an sich bekannter Weise, d.h. beispielsweise durch Heiß-, Kalt-, Heiß-/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden cholesterisch-flüssigkristallinen Zusammensetzungen 10 bis 90 Gew.-%, bevorzugt 20 bis 70 Gew.-% bezogen auf die Gesamtmenge von UV-B und UV-A absorbierenden Substanzen.

Als UV-Filtersubstanzen, die in Kombination mit den erfindungsgemäß zu verwendenden cholesterisch-flüssigkristallinen Zusammensetzungen angewandt werden, kommen beliebige UV-A- und UV-B-Filtersubstanzen in Betracht. Beispielsweise sind zu nennen:

| Nr. | Stoff | CAS-Nr. |
|---|---|---|
| | | (=Säure) |
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino]carbonyl]phenyl]amino]-1,3,5-triazin-2,4-diyl]diimino]bis-,bis(2-ethylhexyl)ester | 154702-15-5 |
| 30 | 2-(2H-Benzotriazol-2-yl)-4-methyl-6-[2-methyl-3-[1,3,3,3-tetramethyl-1-[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexyl)oxy]] (Aniso Triazin) | 187393-00-6 |
| 35 | 2,4,6-Trianilin-(o-carbo-2'-ethylhexyl-1'-oxy)-1,3,5-triazin | 88122-99-0 |

Schließlich sind auch mikronisierte Pigmente wie Titandioxid und Zinkoxid zu nennen.

Zum Schutz menschlicher Haare vor UV-Strahlen können die erfindugsgemäß verwendeten cholesterisch flüssigkristallinen Zusammensetzungen a) und/oder b) in Shampoos, Lotionen, Gelen, Haarsprays, Aerosol-Schaumcremes oder Emulsionen in Konzentrationen von 0,1 bis 20 Gew.-%, bevorzugt 0,5 bis 10 Gew.-%, besonders bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Zusammensetzungen zeichnen sich in der Regel durch ein besonders hohes Reflektionsvermögen im Bereich der UV-A- und UV-B-Strahlung mit scharfer Bandenstruktur aus. Weiterhin lassen sie sich leicht in kosmetische und pharmazeutischen Formulierungen einarbeiten. Außerdem zeichnen sie sich besonders durch ihre hohe Photostabilität, und die damit hergestellten Zubereitungen durch ihr angenehmes Hautgefühl aus.

Die UV-Filterwirkung der erfindungsgemäßen verwendeten cholesterisch flüssigkristallinen Zusammensetzungen a) und/oder b) kann auch zur Stabilisierung von Wirk- und Hilfsstoffen in kosmetischen und pharmazeutischen Formulierungen ausgenutzt werden.

Die folgenden Beispiele sollen die chiralen Dotierstoffe, deren Herstellung und Verwendung näher erläutern.

### Beispiel 1

### Tri-O-acetyl-D-arabino-1,5-anhydro-2-deoxy-hexitol (1)

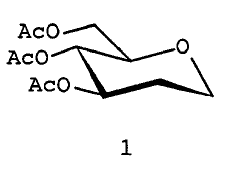

10,0 g (36,5 mmol) 3,4,6-Tri-O-acetyl-D-glucal wurden in 140 ml Ethylacetat/Ethanol (1:1) gelöst, mit einer Spatelspitze Palladiumkatalysator (Pd/C 10%) versetzt und unter einer Wasserstoffatmosphäre bei Raumtemperatur gerührt. Nach vollständiger Hydrierung wurde vom Katalysator abfiltriert und im Vakuum das Lösungsmittelgemisch entfernt.
Ausbeute: 10 g (36,5 mmol, 100%), farbloser Sirup
1H-NMR (400 MHz, CDCl₃): δ = 5,04-4,94 (m, 2H, H-3, H-4), 4,24 (dd, 1H, H-6a), 4,10 (dd, 1H, H-6b), 4,04 (ddd, 1H, H-1eq), 3,56-3,48 (m, 2H, H-1ax, H-5), 2,13-2,07 (m, 4H, H-2eq, 3xH-OAc), 2,04 (s, 3H, 3xH- OAc), 2,03 (s, 3H, 3x H -OAc), 1,83 (mc, 1H, H-2ax) ppm; J_{6a,6b} = 12,4, J_{5,6a} = 5,0, J_{5,6b} = 2,0, J_{1eq,2ax} = 5,0, J_{1eq,2eq} = 1,5 Hz.

### Beispiel 2

### D-arabino-1,5-anhydro-2-deoxy-hexitol (2)

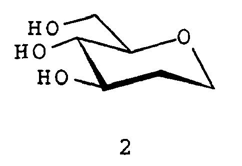

9.5 g (34,7 mmol) 1 wurden in 150 ml wasserfreiem Methanol gelöst und bis zur basischen Reaktion mit Natriummethanolat versetzt. Nach 12 Stunden Rühren bei Raumtemperatur wurde mit saurem Ionenaustauscher (Amberlite IR 120 H⁺-Form**)** neutralisiert, filtriert und das Lösungsmittel im Vakuum entfernt.
Ausbeute: 5,1 g (34,4 mmol, 99%), gelblicher Feststoff
Schmelzpunkt: 76,5-82,2°C
1H-NMR (400 MHz, D₂O): δ = 3,98 (ddd, 1H, H-1eq), 3,89 (dd, 1H, H-6a), 3,70 (dd, 1H, H-6b), 3,66 (mc, 1H, H-3), 3,53 (ddd, 1H, H-1ax), 3,33-2.24 (m, 2H, H-4, H-5), 2,01 (mc, 1H, H-2eq), 1,64 (dddd, 1H, H-2ax) ppm; J_{1eq,1ax} = 12,5, J_{1ax,2ax} = 12,5, J_{1ax,2eq} = 2 0, J_{1eq,2ax} = 5,1, J_{1eq,2eq} = 2,0, J_{2eq,2ax} = 12,5, J_{2ax,3} = 12,5, J_{5,6a} = 2,0, J_{5,6b} = 5,6, J_{6a,6b} = 12,2 Hz.

### Beispiel 3

### (1S,3R,6R,10R)-3-(4'-(4''-Octyloxybenzoyloxy)-phenyl)-10-hydroxy-2,4,7-trioxa-bicyclo[4.4.0]decan (3)

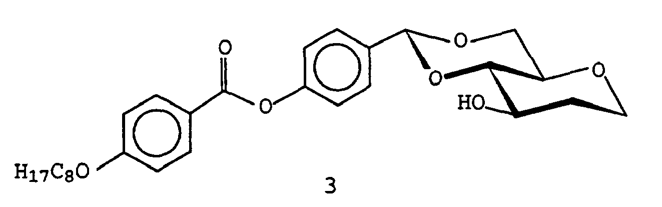

0,91 g (6,0 mmol) 2 und 2,88 g (7,2 mmol) 4-(Octyloxybenzoyloxy)-benzaldehyddimethylacetal wurden in 30 ml wasserfreiem DMF gelöst und mit einer katalytischen Menge 4-Toluolsulfonsäure bis zur sauren Reaktion versetzt. Die Reaktionslösung wurde am Rotationsverdampfer bei 60-65°C und 30 mbar Unterdruck ca. 5 Stunden bewegt. Die Reaktionslösung wurde mit Triethylamin neutralisiert und das DMF im Vakuum entfernt. Das Rohprodukt wurde zweimal aus 50 ml Methanol umkristallisiert und mit 30 ml Petrolether gewaschen.
Ausbeute: 2,61 g (5,4 mmol, 90%), farbloser Feststoff
Schmelzpunkt: 138,8°C.
1H-NMR (400 MHz, C₆D₆): δ = 8,21 (d, 2H, H-2Ar'', H-6Ar''), 7,48 (d, 2H, H-2Ar', H-6Ar'), 7,14 (d, 2H, H-3Ar', H-5Ar'), 6,71(d, 2H, H-3Ar'', H-5Ar''), 5,20 (s, 1H, H-3), 4,13 (dd, 1H, H-5eq), 3,55-3,43 (m, 5H, α-CH₂, H-5ax, H-10, H-8eq), 3,19 (dd, 1H, H-1), 3,06 (ddd, 1H, H-6), 2,98 (ddd, 1H, H-8ax), 2,25 (s, 1H, OH), 1,65-1,53 (m, 2H, H-9eq, H-9ax), 1,53-1,45 (m, 2H, β-CH₂), 1,31-1,10 (m, 10H, 5x CH₂), 0,87 (t, 3H, CH₃) ppm; J_{Ar2'',Ar3''} = 8,7, J_{Ar5'',Ar6''} = 8,7, J_{Ar2',Ar3'} = 8,7, J_{Ar5',Ar6'} = 8,7, J_{5eq,6}= 4,6, J_{5ax,5eq} = 10,2, J_{1,6} = 10,2, J_{1,10} = 10,2, J_{5ax,6} = 10,2, J_{8ax,8eq} = 11,2, J_{8ax,9ax} = 11,2, J_{8ax,9eq} = 4,0, J_{Me,-CH2} = 7,1 Hz.

### Beispiel 4

### (1S,3R,6R,10R)-3-[4'-(4''-Octyloxybenzoyloxy)-phenyl]-10-[4'''-(4''''-octyloxybenzoyloxy)-benzoyloxy]-2,4,7-trioxa-bicyclo[4.4.0]decan (4)

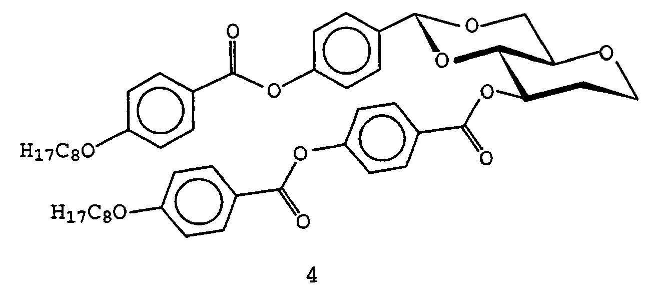

700 mg (1,45 mmol) 3, 535 mg 4-(4-Octyloxybenzoyloxy)-benzoesäure (1,45 mmol), 510 mg DCC (2,47 mmol) und ca. 5 mg 4-(1-Pyrrolidinyl)-pyridin wurden in wasserfreiem Dichlormethan ca. 20 Stunden bei Raumtemperatur gerührt.

Zur Aufarbeitung wurde der Reaktionsansatz im Vakuum zur Trockene eingeengt, der Feststoff in Diethylether aufgenommen, vom unlöslichen Dicyclohexylharnstoff abfiltriert und in ca. 125 ml Ethanol zum Sieden erhitzt. Nach dem Erkalten wurde das so erhaltene Kristallisat abfiltriert, mit EtOH gewaschen und im Vakuum getrocknet.
Ausbeute: 994 mg (1,2 mmol, 82%), farbloser Feststoff
Schmelzpunkt: 180,2°C
1H-NMR (400 MHz, C₆D₆): δ = 8,18-8,12 (m, 6H, H-2Ar'', H-6Ar'', H-2Ar''', H-6Ar''', H-2Ar'''', H-6Ar''''), 7,52 (d, 2H, H-2Ar', H-6Ar'), 7,04 (d, 4H, H-3Ar', H-5Ar', H-3Ar'''', H-5Ar''''), 6,73-6,66 (m, 4H, H-3Ar'', H-5Ar'', H-3Ar''', H-5Ar'''), 5,39 (ddd, 1H, H-10), 5,33 (s, 1H, H-3), 4,14 (dd, 1H, H-5eq), 3,62 (dd, 1H, H-1), 3,55-3,43 (m, 6H, 2x α-CH₂, H-5ax, H-8eq), 3,19 (ddd, 1H, H-6), 2,98 (ddd, 1H, H-8ax), 1,92 (mc, 1H, H-9eq), 1,92 (mc, 1H, H-9ax), 1,50 (sextett, 4H, 2x β-CH₂), 1,31-1,10 (m, 10H, 5x CH₂), 0,83 (t, 6H, 2x CH₃) ppm; J_{Ar2',Ar3'} = 8,7, J_{Ar5',Ar6'} = 8,7, J_{Ar2'''',Ar3''''}= 8,7, J_{Ar5'''',Ar6''''}= 8,7, J_{1,10}= 10,2, J_{9ax,10} = 10,2, J_{9eq,10} = 5,1, J_{5ax,5eq} = 10,2, J_{5eq,6} = 5,1, J_{1,6} = 9,7, J_{8ax,8eq} = 11,2, J_{8ax,9ax} = 11,2, J_{α±cH2},_{β±CH2} = 6,8, J_{β±CH2,γ±CH2} = 6,8, J_{-CH2,-Me} = 7,1 Hz.

### Beispiel 5

### (1S,3R,6R,10R)-3-(4'Methoxyphenyl)-10-hydroxy-2,4,7-trioxa-bicyclo[4.4.0]decan (5)

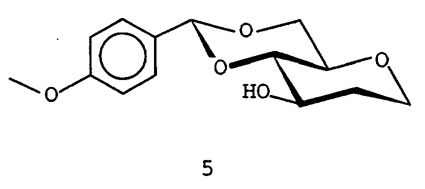

5,0 g (33,8 mmol) 2 und 8,30 g (45,8 mmol) p-Methoxybenzaldehyddimethylacetal wurden in 50 ml wasserfreiem DMA gelöst und mit einer katalytischen Menge 4-Toluolsulfonsäure bis zur sauren Reaktion versetzt. Die Reaktionslösung wurde am Rotationsverdampfer bei 60-65°C und 30 mbar Unterdruck ca. 5 Stunden bewegt. Die Reaktionslösung wurde mit Triethylamin neutralisiert. Man gab 300 ml Essigester hinzu und extrahierte dreimal mit Wasser. Die organische Phase wurde über Na₂SO₄ getrocknet, das Trockenmittel abfiltriert und der Rückstand im Vakuum eingedampft. Das Rohprodukt wurde in wenig Essigester gelöst, mit n-Hexan gefällt, abgesaugt und im Vakuum getrocknet.
Ausbeute: 4,50 g (16,9 mmol, 50%), farbloser Feststoff

### Beispiel 6

### (1S,3R,6R,10R)-3-[4'-Methoxyphenyl]-10-[4''- (Acryloxybutyloxycarbonyloxy)-benzoyloxy]-2,4,7-trioxa-bicyclo[4.4.0]decan (6)

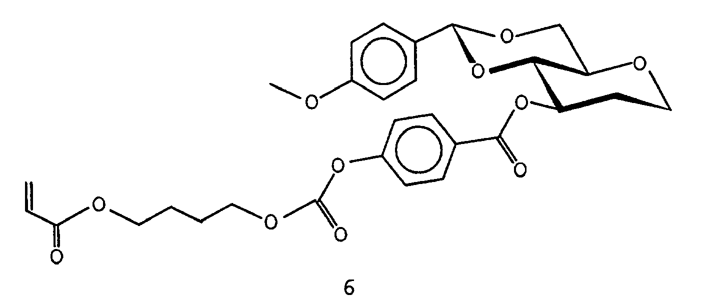

5,0 g (18,8 mmol) 5, 5,79 g 4-Acryloxybutyloxycarbonyloxybenzoesäure (18,8 mmol), 6,60 g DCC (32,0 mmol) und ca. 39 mg 4-(1-Pyrrolidinyl)-pyridin wurden in wasserfreiem Dichlormethan ca. 24 Stunden bei Raumtemperatur gerührt.

Zur Aufarbeitung wurde der Reaktionsansatz im Vakuum zur Trockne eingeengt, der Feststoff in Diethylether aufgenommen und vom unlöslichen Dicyclohexylharnstoff abfiltriert. Das Filtrat wurde eingeengt und durch Säulenchromatographie gereinigt.
Ausbeute: 8,15 g (14,7 mmol, 78%), farbloser Feststoff

### Beispiel 7

### Herstellung cholesterischer Pigmente

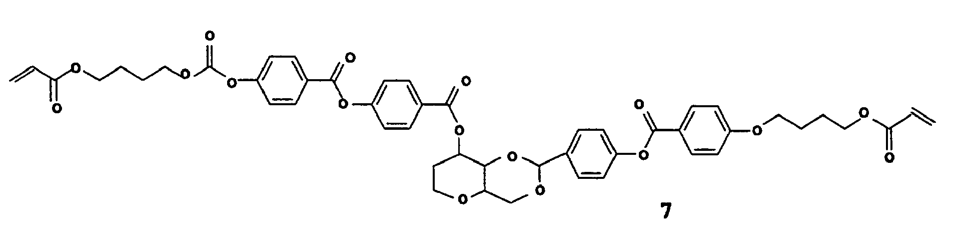

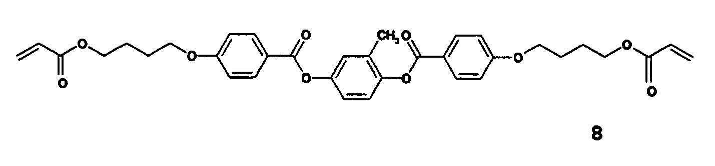

Es wurde eine cholesterisch flüssigkristalline Mischung eingesetzt, die als chirales Monomer eine Verbindung der oben angegebenen Formel 7 und als achirales, nematisches Monomer eine Verbindung der oben angegebenen Formel 8 enthielt. Das unverdünnte cholesterische Gemisch enthielt 94,8 Gew.-% der achiralen, nematischen Verbindung, 5,2 Gew.-% der chiralen Verbindung und als Photoinitiator 2 Gew.-%, bezogen auf die cholesterisch flüssigkristalline Mischung, 1-Hydrocyclohexylphenylketon, das unter der Bezeichnung Irgacure 184 vertrieben wird. Die Mischung zeigte ein λₘₐₓ = 350 nm (T = 70°C).

Zur Herstellung der Pigmente wurde diese Mischung in Methylethylketon gelöst und zur Beschichtung auf eine Polyethylenterephthalfolie aufgetragen. Die Beschichtung erfolgte nach einem in DE-A 19 63 8797 beschriebenen Verfahren.

Die Dicke der cholesterischen Schicht betrug 2,5 µm. Nach Abdampfen des Lösungsmittels bei 70°C wurde die Schicht durch UV-Bestrahlung vernetzt und ausgehärtet. Die so erhaltene gehärtete Cholesterenschicht wurde vom Träger abgelöst und durch Vermahlen und anschließendes Sieben klassiert. Die Korngröße der Pigmentpartikel lag im Bereich < 50 µm.

### Zubereitungen

### Beispiel 8

### Zusammensetzung für die Lippenpflege

| Massengehalt | |
|---|---|
| (Gew.-%) | |
| ad 100 | Eucerinum anhydricum |
| 10,00 | Glycerin |
| 5,00 | Pigment (Beispiel 7) |
| 8,00 | Octyl Methoxycinnamat |
| 5,00 | Zink Oxid |
| 4,00 | Castoröl |
| 4,00 | Pentaerythrithyl Stearat/Caprat/Caprylat Adipat |
| 3,00 | Glyceryl Stearat SE |
| 2,00 | Bienenwachs |
| 0,50 | Tocopheryl Acetat |
| 2,00 | Microkristallines Wachs |
| 2,00 | Quaternium-18 Bentonit |
| 1,50 | PEG-45/Dodecyl Glycol Copolymer |

### Beispiel 9

### Zusammensetzung für Sunblocker mit Mikropigmenten

| Massengehalt | |
|---|---|
| (Gew.-%) | |
| ad 100 | Wasser |
| 10,00 | Octyl Methoxcinnamat |
| 6,00 | PEG-7-Hydrogenated Castor Öl |
| 6,00 | Titanium Dioxid |
| 5,00 | Pigment (Beispiel 7) |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 10

### Fettfreies Gel

| Massengehalt | |
|---|---|
| (Gew.-%) | |
| ad 100 | Wasser |
| 8,00 | Octyl Methoxycinnamat |
| 5,00 | Pigment (Beispiel 7) |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 11

### Sonnencreme (LSF 20)

| Massengehalt | |
|---|---|
| (Gew.-%) | |
| ad 100 | Wasser |
| 8,00 | Octyl Methoxycinnamat |
| 6,00 | PEG-7-Hydrogenated Castor Öl |
| 5,00 | Pigment (Beispiel 7) |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 12

### Sonnencreme wasserfest

| Massengehalt | |
|---|---|
| (ges.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Pigment (Beispiel 7) |
| 4,00 | Glycerin |
| 3,00 | Jojoba Öl |
| 2,00 | 4-Methylbenzyliden Campher |
| 1,50 | PEG-45/Dodecyl Glycol Copolymer |
| 1,50 | Dimethicon |
| 0,70 | Magnesium Sulfat |
| 0,50 | Magnesium Stearat |
| 0,50 | Tocopheryl Acetat |
| 0,15 | Fragrance |

### Beispiel 13

### Sonnenmilch (LSF 6)

| Massengehalt | |
|---|---|
| (Gew.-%) | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 3,00 | Pigment (Beispiel 7) |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |

## Patentansprüche

1. Verwendung von Verbindungen der allgemeinen Formel I, 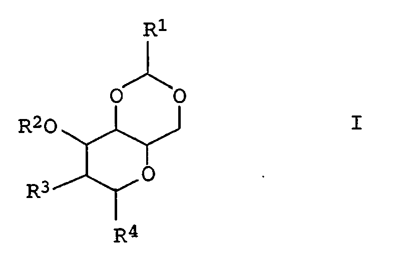 in der die Substituenten unabhängig voneinander folgende Bedeutung haben:
R¹ Z¹-Y¹-(A¹)ₘ-Y²-M¹-Y³-(A²)ₙ-Y⁴-;
R² Wasserstoff, C₁-C₁₂-Alkyl, C₁-C₁₂-Alkylcarbonyl, Aryl, Arylcarbonyl, Z²-Y⁵-(A³)ₒ-Y⁶-M²-Y⁷-(A⁴)ₚ-Y¹¹-;
R³ Wasserstoff, C₁-C₁₂-Alkyl, Aryl, OR⁵;
R⁴ Wasserstoff, C₁-C₁₂-Alkyl, Aryl, OR⁶;
R⁵ und R⁶ Wasserstoff, C₁-C₁₂-Alkyl, C₁-C₁₂-Alkylcarbonyl, Aryl, Arylcarbonyl, Z³-Y⁸-(A⁵)_{q}-Y⁹-M³-Y¹⁰-(A⁶)ᵣ-Y¹²-;
A¹ bis A⁶ ein Spacer einer Kettenlänge von 1 bis 30 C-Atomen;
M¹ bis M³ eine mesogene Gruppe;
Y¹ bis Y¹⁰ eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R)- oder -(R)N-C(**=**O)-, -CH₂-O-, -O-CH₂-, -CH**=**N-, -N**=**CH- oder -N=N-;
Y¹¹ und Y¹² eine chemische Bindung, -C(=O)-, -O-C(**=**O)-, -CH**=**CH-C(**=**O)-, -(R)N-C(**=**O)-, -CH₂-, -O-CH₂-;
R Wasserstoff, C₁-C₄-Alkyl;
Z¹ bis Z³ Wasserstoff, C₁-C₁₂-Alkyl, eine polymerisierbare Gruppe oder ein Rest, der eine polymerisierbare Gruppe trägt;
m bis r 0 oder 1,
wobei die Reste A¹ bis A⁶, Y¹ bis Y¹⁰, Y¹¹ und Y¹² sowie Z¹ bis Z³ gleich oder verschieden sein können, und wobei für den Fall, daß einer oder mehrere der Indices m bis r gleich null sind, mindestens einer der jeweils von A benachbarten Reste Y eine chemische Bindung bedeutet,
als chirale Dotierstoffe für nematische oder cholesterische Flüssigkristalle zur Erzeugung farbig, im UV- oder IR-Bereich reflektierender Schichten oder zur Herstellung von flüssigkristallin cholesterisch geordneten Pigmenten.

2. Verwendung nach Anspruch 1, in der die Substituenten folgende Bedeutung haben:
R¹ Z¹-Y¹-(A¹)ₘ-Y²-M¹-Y³-(A²)ₙ-Y⁴-;
R² Z²-Y⁵-(A³)ₒ-Y⁶-M²-Y⁷-(A⁴)ₚ-Y¹¹-;
R³ und R⁴ Wasserstoff;
A¹ und A³ ein Spacer einer Kettenlänge von 1 bis 6 C-Atomen;
Y¹ bis Y⁷ eine chemische Bindung, -O-, -S-, -C(=O)-, -C(**=**O)-O-, -O-C(**=**O)-;
Y¹¹ eine chemische Bindung, -C(=O)-, -O-C(**=**O)-;
M¹ und M² ein mesogener Rest aus der Gruppe, bestehend aus: 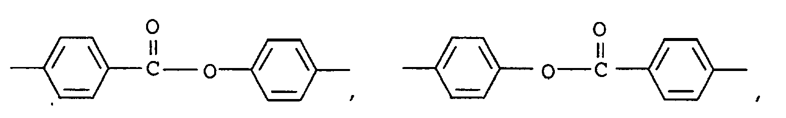 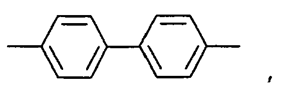 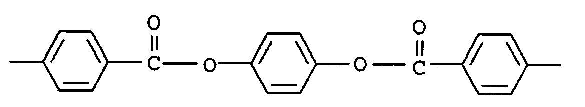 und 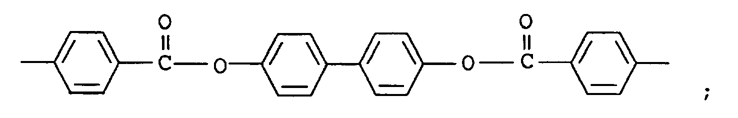
Z¹ und Z² Wasserstoff, C₄-C₁₀-Alkyl, eine polymerisierbare Gruppe oder ein Rest, der eine polymerisierbare Gruppe trägt;
m 0 oder 1;
n 0;
o 0 oder 1;
p 0,
wobei mindestens einer der Reste Y³ und Y⁴ bzw. Y⁷ und Y¹¹ eine chemische Bindung bedeutet.

3. Verwendung von Verbindung Ia nach einem der Ansprüche 1 oder 2, 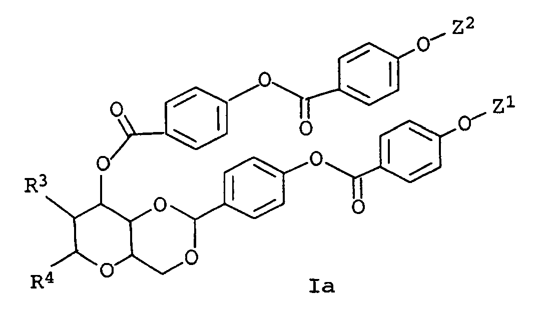 in der die Substituenten unabhängig voneinander folgende Bedeutung haben:
R³ Wasserstoff;
R⁴ Wasserstoff;
Z¹ und Z² Wasserstoff, C₄-C₁₀-Alkyl, eine polymerisierbare Gruppe oder ein Rest, der eine polymerisierbare Gruppe trägt.

4. Verwendung nach einem der Ansprüche 1 bis 3, **dadurch gekennzeichnet, daß** mindestens einer der Reste Z¹ bis Z³ eine polymerisierbare Gruppe oder einen Rest, der eine polymerisierbare Gruppe enthält, bedeutet.

5. Chirale Dotierstoffe der allgemeinen Formel Ib, 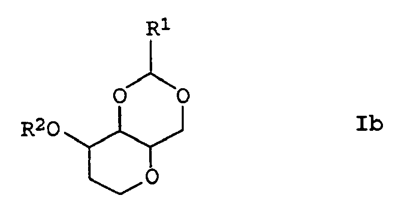 in der die Substituenten unabhängig voneinander folgende Bedeutung haben:
R¹ Z¹-Y¹-(A¹)ₘ-Y²-M¹-Y³-(A²)ₙ-Y⁴-;
R² Wasserstoff, C₁-C₁₂-Alkyl, C₁-C₁₂-Alkylcarbonyl, Aryl, Arylcarbonyl, Z²-Y⁵-(A³)ₒ-Y⁶-M²-Y⁷-(A⁴)ₚ-Y¹¹-_{;}
A¹ bis A⁴ ein Spacer einer Kettenlänge von 1 bis 30 C-Atomen;
M¹ und M² eine mesogene Gruppe;
Y¹ bis Y⁷ eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R)- oder -(R)N-C(**=**O)-, -CH₂-O-, -O-CH₂-, -CH**=**N-, -N**=**CH- oder -N**=**N-;
Y¹¹ eine chemische Bindung, -C(=O)-, -O-C(**=**O)-, -CH**=**CH-C(**=**O)-, -(R)N-C(**=**O)-, -CH₂-, -O-CH₂-;
R Wasserstoff, C₁-C₄-Alkyl;
Z¹ und Z² Wasserstoff, C₁-C₁₂-Alkyl, eine polymerisierbare Gruppe oder ein Rest, der eine polymerisierbare Gruppe trägt;
m bis p 0 oder 1,
wobei die Reste A¹ bis A⁴, Y¹ bis Y⁷ sowie Z¹ und Z² gleich oder verschieden sein können, und wobei für den Fall, daß einer oder mehrere der Indices m bis p gleich null sind, mindestens einer der jeweils von A benachbarten Reste Y eine chemische Bindung bedeutet.

6. Chirale Dotierstoffe nach Anspruch 5, in der die Substituenten folgende Bedeutung haben:
R¹ Z¹-Y¹-(A¹)ₘ-Y²-M¹-Y³-(A²)ₙ-Y⁴-;
R2 Z²-y⁵-(A³)ₒ-Y⁶-M²-Y⁷-(A⁴)ₚ-Y¹¹-_{;}
A¹ und A³ ein Spacer einer Kettenlänge von 1 bis 6 C-Atomen;
Y¹ bis Y⁷ eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R)- oder -(R)N-C(**=**O)-, -CH₂-O-, -O-CH₂-, -CH**=**N-, -N**=**CH- oder -N=N-;
Y¹¹ eine chemische Bindung, -C(=O)-, -O-C(**=**O)-, -CH**=**CH-C(**=**O)-, -(R)N-C(**=**O)-, -CH₂-, -O-CH₂-;
M¹ und M² ein mesogener Rest aus der Gruppe, bestehend aus: 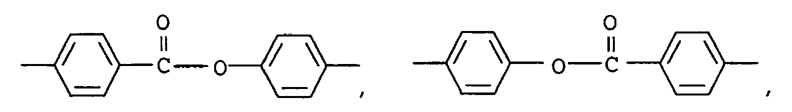 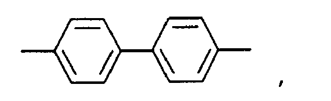 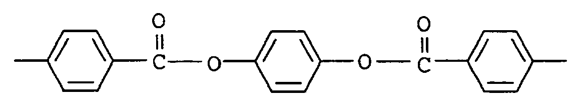 und 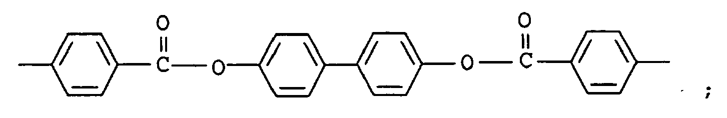
Z¹ und Z² Wasserstoff, C₄-C₁₀-Alkyl, eine polymerisierbare Gruppe oder ein Rest, der eine polymerisierbare Gruppe trägt;
m 0 oder 1;
n 0;
o 0 oder 1;
p 0,
wobei mindestens einer der Reste Y³ und Y⁴ bzw. Y⁷ und Y¹¹ eine chemische Bindung bedeutet.

7. Chirale Dotierstoffe nach einem der Ansprüche 5 oder 6 mit der allgemeinen Formel Ia, 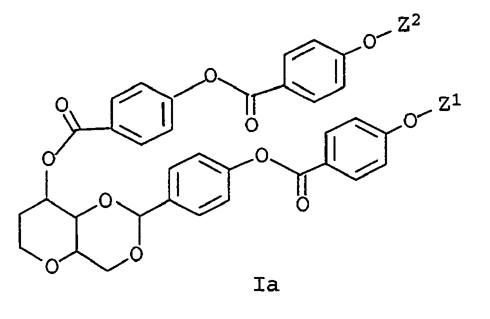 in der die Substituenten unabhängig voneinander folgende Bedeutung haben:
Z¹ und Z² Wasserstoff, C₄-C₁₀-Alkyl, eine polymerisierbare Gruppe oder ein Rest, der eine polymerisierbare Gruppe trägt.

8. Cholesterisch-flüssigkristalline Zusammensetzungen, enthaltend
a) mindestens ein chirales flüssigkristallines Monomeres der allgemeinen Formel I, gemäß Anspruch 1 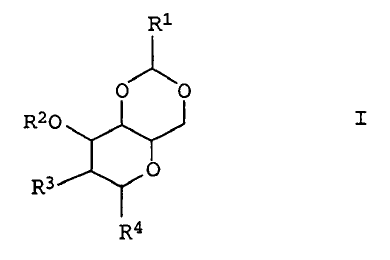 oder
b) eine Mischung aus
b₁) mindestens einem achiralen flüssigkristallinen polymerisierbaren Monomeren der allgemeinen Formel II
Z⁴-Y¹³-(A⁷)ₛ-Y¹⁴-M⁴-Y¹⁵⁻(A⁸)ₜ₋Y¹⁶-Z⁵ II
in der die Variablen unabhängig voneinander folgende Bedeutung haben:
A⁷ und A⁸ ein Spacer einer Kettenlänge von 1 bis 30 C-Atomen;
M⁴ eine mesogene Gruppe;
Y¹³ bis Y¹⁶ eine chemische Bindung, -O-, -S-, -C(=O)-, -C(**=**O)-O-, -O-C(**=**O)-, -CH**=**CH-C(**=**O)-O-, -O-C(**=**O)-O-, -C(**=**O)-N(R⁷)- oder -(R⁷)N-C(**=**O)-, -CH₂-O-, -O-CH₂-, -CH**=**N-, -N**=**CH- oder -N**=**N-;
R⁷ Wasserstoff, C₁-C₄-Alkyl;
s 0 oder 1;
t 0 oder 1;
Z⁴ und Z⁵ Wasserstoff, C₁-C₁₂-Alkyl, eine polymerisierbare Gruppe oder ein Rest, der eine polymerisierbare Gruppe trägt,
wobei die Reste A⁷ und A⁸ sowie Y¹³ bis Y¹⁶ gleich oder verschieden sein können, mindestens eine der Variablen Z⁴ oder Z⁵ eine polymerisierbare Gruppe oder einen Rest, der eine polymerisierbare Gruppe trägt, darstellt und für den Fall, daß einer oder beide der Indices s und t gleich null sind, mindestens einer der Reste Y¹³ und Y¹⁴ bzw. Y¹⁵ und Y¹⁶ eine chemische Bindung bedeutet, und
b₂) mindestens ein chirales flüssigkristallines Monomer der allgemeinen Formel I, gemäß Anspruch 1.

9. Cholesterisch-flüssigkristalline Zusammensetzungen nach Anspruch 8, enthaltend als chirales flüssigkristallines Monomer a) oder b₂) mindestens eine chirale Verbindung der allgemeinen Formel Ia, 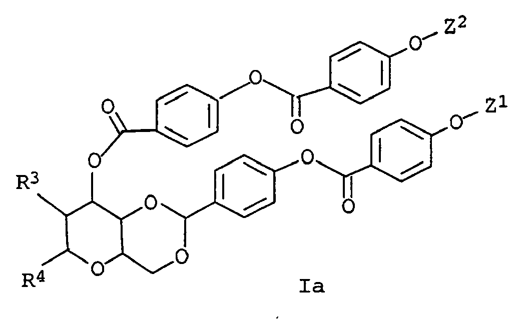 in der die Substituenten unabhängig voneinander folgende Bedeutung haben:
R³ Wasserstoff;
R⁴ Wasserstoff;
Z¹ und Z² Wasserstoff, C₄-C₁₀-Alkyl, eine polymerisierbare Gruppe oder ein Rest, der eine polymerisierbare Gruppe trägt.

10. Cholesterisch-flüssigkristalline Zusammensetzungen nach einem der Ansprüche 8 oder 9, **dadurch gekennzeichnet, daß** sie im UV-Bereich linkszirkular polarisiertes Licht reflektieren.

11. Verwendung von cholesterisch-flüssigkristallinen Zusammensetzungen, definiert gemäß einem der Ansprüche 8 bis 10, als UV-Filter zur Herstellung von kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

12. Verwendung von cholesterisch-flüssigkristallinen Zusammensetzungen nach Anspruch 11 zur Herstellung von als photostabilen UV-Reflektoren.

13. Verwendung von cholesterisch-flüssigkristallinen Zusammensetzungen, definiert gemäß einem der Ansprüche 8 bis 10, als UV-Stabilisatoren zur Herstellung von kosmetischen und pharmazeutischen Formulierungen.

14. Verwendung von cholesterisch-flüssigkristallinen Zusammensetzungen nach einem der Ansprüchen 11 bis 13 in Form von Pigmenten.

15. Pigmente enthaltend cholesterisch-flüssigkristalline Zusammensetzungen definiert gemäß einem der Ansprüche 8 bis 10.

16. Pigmente nach Anspruch 15, **dadurch gekennzeichnet, daß** es sich um Mehrschichtpigmente handelt.

17. Kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten, im UV-Bereich absorbierenden Verbindungen, als photostabile UV-Filter wirksame Mengen von cholesterisch-flüssigkristallinen Zusammensetzungen, definiert gemäß einem der Ansprüche 8 bis 10, enthalten.

18. Kosmetische und pharmazeutische Zubereitungen nach Anspruch 17, enthaltend als UV-Filter cholesterisch-flüssigkristalline Zusammensetzungen in Form von Pigmenten.

19. Kosmetische und pharmazeutische Zubereitungen nach einem der Ansprüche 17 oder 18, enthaltend als UV-Filter cholesterisch-flüssigkristalline Zusammensetzungen in Form von mehrschichtigen Pigmenten.

## Claims

1. The use of compounds of the general formula I 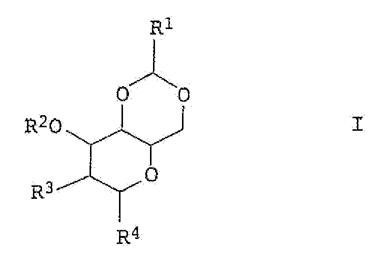 in which the substituents, independently of one another, have the following meanings:
R¹ is Z¹-Y¹-(A¹)ₘ-Y²-M¹-Y³-(A²)ₙ₋Y⁴-;
R² is hydrogen, C₁-C₁₂-alkyl, C₁-C₁₂-alkylcarbonyl, aryl, arylcarbonyl or Z²-Y⁵-(A³)ₒ-Y⁶-M²-Y⁷-(A⁴)ₚ-Y¹¹-;
R³ is hydrogen, C₁-C₁₂-alkyl, aryl or OR⁵;
R⁴ is hydrogen, C₁-C₁₂-alkyl, aryl or OR⁶;
R⁵ and R⁶ are hydrogen, C₁-C₁₂-alkyl, C₁-C₁₂-alkylcarbonyl, aryl, arylcarbonyl or Z³-Y⁸-(A⁵)_{q}-Y⁹-M³-Y¹⁰-(A⁶)ᵣ-Y¹²-;
A¹ to A⁶ spacers having a chain length of from 1 to 30 carbon atoms;
M¹ to M³ are mesogenic groups;
Y¹ to Y¹⁰ are chemical bonds, -O-, -S-, -C(=O)-, -C(=O)-O-, -O-C(=O)-, -CH=CH-C(=O)-O-, -O-C(=O)-O-, -C(=O)-N(R)- or -(R)N-C(=O)-, -CH₂-O-, -O-CH₂-, -CH=N-, -N=CH- or -N=N-;
Y¹¹ and Y¹² are chemical bonds, -C(=O)-, -O-C(=O)-, -CH=CH-C(=O)-, -(R)N-C(=O)-, -CH₂- or -O-CH₂-;
R is hydrogen or C₁-C₄-alkyl;
Z¹ to Z³ are hydrogen, C₁-C₁₂-alkyl, polymerizable groups or radicals carrying polymerizable groups;
m to r are 0 or 1,
where the radicals A¹ to A⁶, Y¹ to Y¹⁰, Y¹¹ and Y¹² and Z¹ to Z³ may be identical or different, and where, in the case where one or more of the indices m to r are zero, at least one of the radicals Y in each case adjacent to A is a chemical bond,
as chiral dopants for nematic or cholesteric liquid crystals for the generation of layers which reflect in color in the UV or IR region or for the preparation of pigments having a liquid-crystalline, cholesteric order.

2. The use as claimed in claim 1, in which the substituents have the following meanings:
R¹ is Z¹-Y¹-(A¹)ₘ-Y²-M¹-Y³-(A²)ₙ-Y⁴⁻;
R² is Z²-Y⁵-(A³)ₒ-Y⁶-M²-Y⁷-(A⁴)ₚ-Y¹¹-;
R³ and R⁴ are hydrogen;
A¹ and A³ are spacers having a chain length of from 1 to 6 carbon atoms;
Y¹ to Y⁷ are chemical bonds, -O-, -S-, -C(=O)-, -C(=C)-O-, -O-C(=O)-;
Y¹¹ is a chemical bond, -C(=O)- or -O-C(=O)-;
M¹ and M² are mesogenic radicals from the group consisting of: 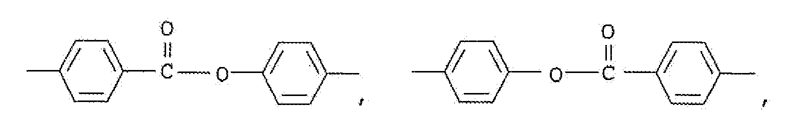 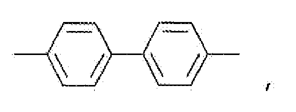 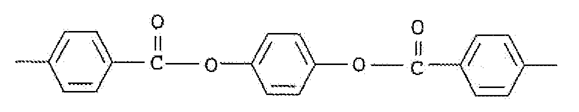 and 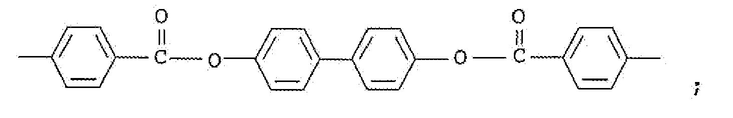
Z¹ and Z² are hydrogen, C₄-C₁₀-alkyl, polymerizable groups or radicals carrying polymerizable groups;
m is 0 or 1;
n is 0;
o is 0 or 1; and
p is 0,
where at least one of the radicals Y³ and Y⁴ or Y⁷ and Y¹¹ is a chemical bond.

3. The use of a compound Ia as claimed in one of claims 1 and 2 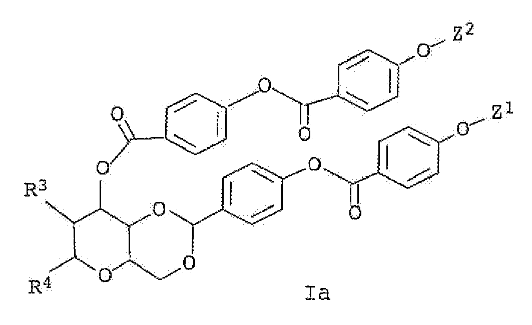 in which the substituents, independently of one another, have the following meanings:
R³ is hydrogen;
R⁴ is hydrogen;
Z¹ and Z² are hydrogen, C₄-C₁₀-alkyl, polymerizable groups or radicals carrying polymerizable groups.

4. The use as claimed in one of claims 1 to 3, wherein at least one of the radicals Z¹ to Z³ is a polymerizable group or a radical containing a polymerizable group.

5. A chiral dopant of the general formula Ib 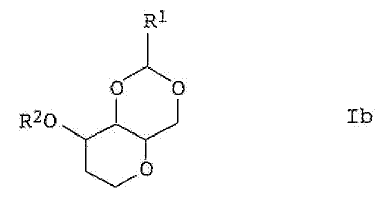 in which the substituents, independently of one another, have the following meanings:
R¹ is Z¹-Y¹-(A¹)ₘ-Y²-M¹-Y³-(A²)ₙ-Y⁴-;
R² is hydrogen, C₁-C₁₂-alkyl, C₁-C₁₂-alkylcarbonyl, aryl, arylcarbonyl or Z²-Y⁵-(A³)ₒ-Y⁶-M²-Y⁷-(A⁴)ₚ-Y¹¹-;
A¹ to A⁴ are spacers having a chain length of from 1 to 30 carbon atoms;
M¹ and M² are mesogenic groups;
Y¹ to Y⁷ are chemical bonds, -O-, -S-, -C(=O)-, -C(=O)-O-, -O-C(=O)-, -CH=CH-C(=O)-O-, -O-C(=O)-O-, -C(=O)-N(R)- or -(R)N-C(=O)-, -CH₂-O-, -O-CH₂-, -CH=N-, -N=CH- or -N=N-;
Y¹¹ is a chemical bond, -C(=O)-, -O-C(=O)-, -CH=CH-C(=O)-, -(R)N-C(=O)-, -CH₂- or -O-CH₂-;
R is hydrogen or C₁-C₄-alkyl;
Z¹ and Z² are hydrogen, C₁-C₁₂-alkyl, polymerizable groups or radicals carrying polymerizable groups;
m to p are 0 or 1,
where the radicals A¹ to A⁴, Y¹ to Y⁷ and Z¹ and Z² may be identical or different, and where, in the case where one or more of the indices m to p are zero, at least one of the radicals Y in each case adjacent to A is a chemical bond.

6. A chiral dopant as claimed in claim 5, in which the substituents have the following meanings:
R¹ is Z¹-Y¹-(A¹)ₘ-Y²-M¹-Y³-(A²)ₙ-Y⁴;
R² is Z²-Y⁵-(A³)ₒ-Y⁶-M²-Y⁷-(A⁴)ₚ-Y¹¹;
A¹ and A³ are spacers having a chain length of from 1 to 6 carbon atoms;
Y¹ to Y⁷ are chemical bonds, -O-, -S-, -C(=O)-, -C(=O)-O-, -O-C(=O)-, -CH=CH-C(=O)-O-, -O-C(=O)-O-, -C(=O)-N(R)- or -(R)N-C(=O)-, -CH₂-O-, -O-CH₂-, -CH=N-, -N=CH- or -N=N-;
Y¹¹ is a chemical bond, -C(=O)-, -O-C(=O)-, -CH=CH-C(=O)-, -(R)N-C(=O)-, -CH₂- or -O-CH₂-;
M¹ and M² are mesogenic radicals from the group consisting of: 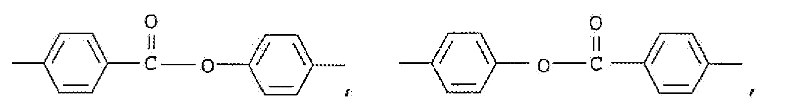 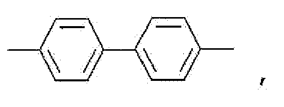 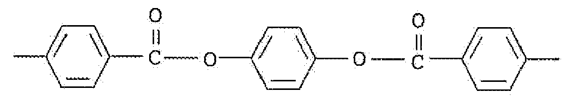 and 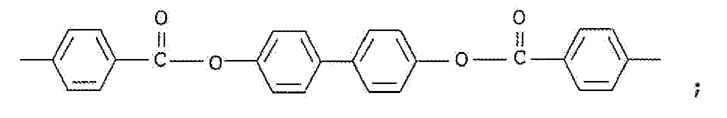
Z¹ and Z² are hydrogen, C₄-C₁₀-alkyl, polymerizable groups or radicals carrying polymerizable groups;
m is 0 or 1;
n is 0;
o is 0 or 1;
p is 0,
where at least one of the radicals Y³ and Y⁴ or Y⁷ and Y¹¹ is a chemical bond.

7. A chiral dopant as claimed in one of claims 5 and 6, of the general formula Ia, 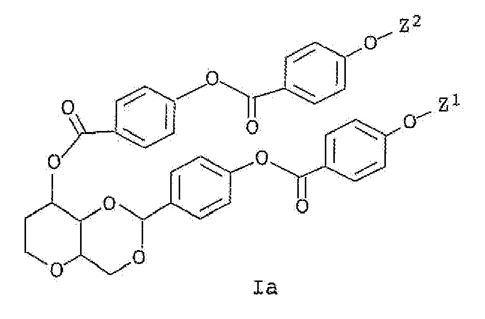 in which the substituents, independently of one another, have the following meanings:
Z¹ and Z² are hydrogen, C₄-C₁₀-alkyl, polymerizable groups or radicals carrying polymerizable groups.

8. A cholesteric liquid-crystalline composition comprising
a) at least one chiral liquid-crystalline monomer of the general formula I as claimed in claim 1 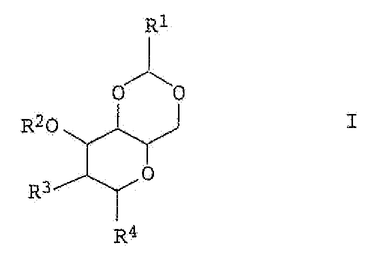 or
b) a mixture of
b₁) at least one achiral, liquid-crystalline, polymerizable monomer of the general formula II
Z⁴-Y¹³-(A⁷)ₛ-Y¹⁴-M⁴-Y¹⁵-(A⁹)ₜ-Y¹⁶-Z⁵ II
in which the variables, independently of one another, have the following meanings:
A⁷ and A⁸ are spacers having a chain length of from 1 to 30 carbon atoms;
M⁴ is a mesogenic group;
Y¹³ to Y¹⁶ are chemical bonds, -O-, -S-, -C(=O)-, -C(=O)-O-, -O-C(=O)-, -CH=CH-C(=O)-O-, -O-C(=O)-O-, -C(=D)-N(R⁷)- or -(R⁷)N-C(=O)-, -CH₂-O-, -O-CH₂-, -CH=N-, -N=CH- or -N=N-;
R⁷ is hydrogen or C₁-C₄-alkyl;
s is 0 or 1;
t is 0 or 1;
Z⁴ and Z⁵ are hydrogen, C₁-C₁₂-alkyl, polymerizable groups or radicals carrying polymerizable groups,
where the radicals A⁷ and A⁸ and Y¹³ to Y¹⁶ may be identical or different, at least one of the variables Z⁴ or Z⁵ is a polymerizable group or a radical carrying a polymerizable group, and, in the case where one or both of the indices s and t are zero, at least one of the radicals Y¹³ and Y¹⁴ or Y¹⁵ and Y¹⁶ is a chemical bond, and
b₂) at least one chiral liquid-crystalline monomer of the general formula I as claimed in claim 1.

9. A cholesteric liquid-crystalline composition as claimed in claim 8, comprising, as chiral liquid-crystalline monomer a) or b₂), at least one chiral compound of the general formula Ia, 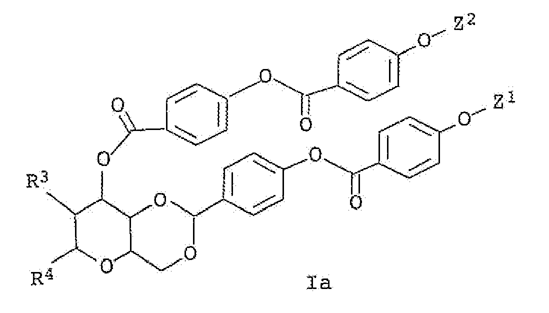 in which the substituents, independently of one another, have the following meanings:
R³ is hydrogen;
R⁴ is hydrogen;
Z¹ and Z² are hydrogen, C₄-C₁₀-alkyl, polymerizable groups or radicals carrying polymerizable groups.

10. A cholesteric liquid-crystalline composition as claimed in one of claims 8 and 9, which deflects left-handed circular-polarized light in the UV region.

11. The use of a cholesteric liquid-crystalline composition as defined in one of claims 8 to 10 as a UV filter in the manufacture of cosmetic and pharmaceutical preparations for protecting the human skin or human hair against sunlight, alone or together with UV-absorbent compounds which are known per se for cosmetic and pharmaceutical preparations.

12. The use of a cholesteric liquid-crystalline composition as claimed in claim 11 in the manufacture of a photostable UV reflector.

13. The use of a cholesteric liquid-crystalline composition as defined in one of claims 8 to 10 as a UV stabilizer in the manufacture of cosmetic and pharmaceutical formulations.

14. The use of a cholesteric liquid-crystalline composition as claimed in one of claims 11 to 13 in the form of a pigment.

15. A pigment comprising a cholesteric liquid-crystalline composition as defined in one of claims 8 to 10.

16. A pigment as claimed in claim 15, which is a multilayer pigment.

17. A cosmetic or pharmaceutical preparation for protecting the human epidermis or human hair against UV light in the region from 280 to 400 nm, which comprises, alone or together with UV-absorbent compounds known per se for cosmetic and pharmaceutical preparations, an amount effective at a photostable UV filter of a cholesteric liquid-crystalline composition as defined in one of claims 8 to ID in a cosmetically or pharmaceutically suitable excipient.

18. A cosmetic or pharmaceutical preparation as claimed in claim 17 comprising, as UV filter, a cholesteric liquid-crystalline composition in the form of a pigment.

19. A cosmetic or pharmaceutical preparation as claimed in one of claims 17 and 18 comprising, as UV filter, a cholesteric liquid-crystalline composition in the form of a multilayered pigment.

## Revendications

1. Utilisation de composés de la formule générale I : 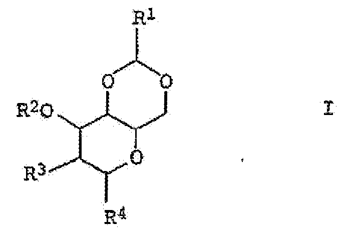 dans laquelle les substituants ont, indépendamment les uns des autres, la signification, suivante :
R¹ représente Z¹-Y¹-(A¹)ₘ-Y²-M¹-Y³-(A²)ₙ-Y⁴ ;
R² représente de l'hydrogène, un alkyle en C₁-C₁₂, un alkylcarbonyle en C₁-C₁₂, un aryle, un arylcarbonyle, Z²-Y⁵-(A³)ₒ-Y⁶-M²-Y⁷-(A⁴)ₚ-Y¹¹ ;
R³ représente de l'hydrogène, un alkyle en C₁-C₁₂, un aryle, OR⁵ ;
R⁴ représente de l'hydrogène, un alkyle en C₁-C₁₂, un aryle, OR⁶ ;
R⁵ et R⁶ représentent de l'hydrogène, un alkyle en C₁-C₁₂, un alkylcarbonyle en C₁-C₁₂, un aryle, un arylcarbonyle, Z³-Y⁸-(A⁵)_{q}-Y⁹-M³-Y¹⁰-(A⁶)ᵣ-Y¹² ;
A¹ à A⁶ représentent un radical espaceur d'une longueur de chaîne de 1 à 30 atomes de carbone ;
M¹ à M³ représentent un groupe mésogène ;
Y¹ à Y¹⁰ représentent une liaison chimique, -O-, -S-, -C(=O)-, -C(=O)-O-, -O-C(=O)-, -CH=CH-C(=O)-O-, -O-C(=O)-O-, -C(=O)-N(R)- ou -(R)N-C(=O)-, -CH₂-O-, -O-CH₂-, -CH=N-, -N=CH- ou -N=N- ;
Y¹¹ et Y¹² représentent une liaison chimique, -C(=O)-, -O-C(=O)-, -CH=CH-C(=O)-, -(R)N-C(=O)-, -CH₂-, -O-CH₂- ;
R représente de l'hydrogène, un alkyle en C₁-C₄ ;
Z¹ à Z³ représentent de l'hydrogène, un alkyle en C₁-C₁₂, un groupe polymérisable ou un radical qui porte un groupe polymérisable ;
m à r valent 0 ou 1,
les radicaux A¹ à A⁶, Y¹ à Y¹⁰, Y¹¹ et Y¹², ainsi que les radicaux Z¹ à Z³, pouvant être identiques ou différents et, dans le cas où un ou plusieurs des indices m à r valent zéro, au moins l'un des radicaux Y, respectivement voisins de A représentant une liaison chimique,
comme substances de dopage chirales pour des cristaux liquides nématiques ou cholestériques dans le but de produire des couches colorées et réfléchissantes dans les plages des U.V. ou des IR, ou pour la fabrication de pigments à base de cristaux liquides d'ordre cholestérique.

2. Utilisation selon la revendication 1, dans laquelle les substituants ont 1a signification suivante :
R¹ représente Z¹-Y¹-(A¹)ₘ-Y²-M¹-Y³-(A²)ₙ-Y⁴ ;
R² représente Z²-Y⁵-(A³)ₒ-Y⁶-M²-Y⁷⁻(A⁴)ₚ-Y¹¹ ;
R³ et R⁴ représentent de l'hydrogène ;
A¹ et A³ représentent un radical espaceur d'une longueur de chaîne de 1 à 6 atomes de carbone ;
Y¹ à Y⁷ représentent une liaison chimique, -O-, -S-, -C(=O)-, -C(=O)-O-, -O-C(=O)- ;
Y¹¹ représente une liaison chimique, -C(=O)-, -O-C(=O)- ;
M¹ et M² représentent un radical mésogène choisi dans le groupe constitué de : 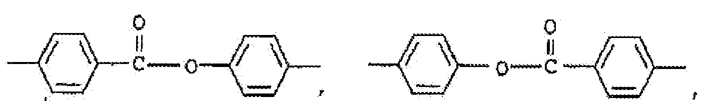 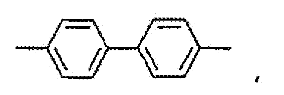 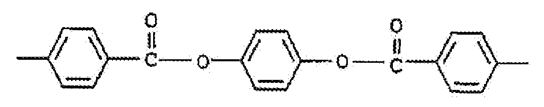 et 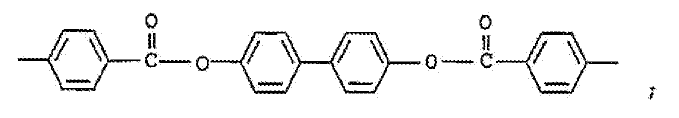
Z¹ et Z² représentent de l'hydrogène, un alkyle en C₄-C₁₀, un groupe polymérisable ou un radical qui porte un groupe polymérisable ;
m vaut 0 ou 1 ;
n vaut 0 ;
o vaut 0 ou 1 ;
p vaut 0,
au moins l'un des radicaux Y³ et Y⁴ ou Y⁷ et Y¹¹ représentant une liaison chimique.

3. Utilisation du composé la selon l'une quelconque des revendications 1 ou 2, 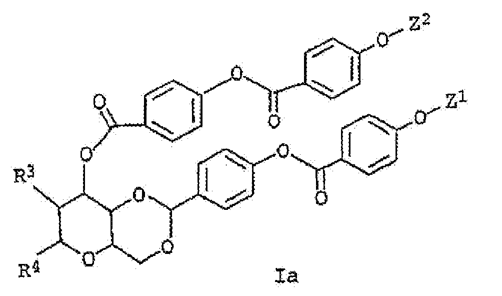 dans laquelle les substituants ont, indépendamment les uns des autres, la signification suivante :
R³ représente de l'hydrogène ;
R⁴ représente de l'hydrogène ;
Z¹ et Z² représentent de l'hydrogène, un alkyle en C₄-C₁₀), un groupe polymérisable ou un radical qui porte un groupe polymérisable.

4. Utilisation selon l'une quelconque des revendications 1 à 3, **caractérisée en ce qu'**au moins l'un des radicaux Z¹ à Z³ représente un groupe polymérisable ou un radical qui contient un groupe polymérisable.

5. Substances de dopage chirales de la formule générale Ib : 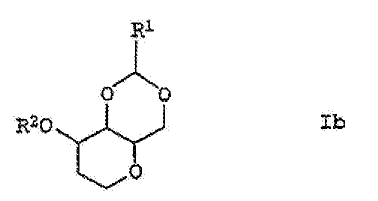 dans laquelle les substituants ont, indépendamment les uns des autres, la signification suivante :
R¹ représente Z¹-Y¹-(A¹)ₘ-Y²-M¹-Y³-(A²)ₙ-Y⁴ ;
R² représente de l'hydrogène, un alkyle en C₁-C₁₂, un alkylcarbonyle en C₁-C₁₂, un aryle, un arylcarbonyle, Z²-Y⁵-(A³)ₒ-Y⁶-M²-Y⁷-(A⁴)ₚ-Y¹¹- ;
A¹ à A⁴ représentent un radical espaceur d'une longueur de chaîne de 1 à 30 atomes de carbone ;
M¹ et M² représentent un groupe mésogène ;
Y¹ à Y⁷ représentent une liaison chimique, -O-, -S-, -C(=O)-, -C(=O)-O-, -O-C(=O)-, -CH=CH-C(=O)-O-, -O-C(=O)-O-, -C(=O)-N(R)- ou -(R)N-C(=O)-, -CH₂-O-, -O-CH₂-, -CH=N-, -N=CH- ou -N=N- ;
Y¹¹ représente une liaison chimique, -C(=O)-, -O-C(=O)-, -CH=CH-C(=O)-, -(R)N-C(=O)-, -CH₂-, -O-CH₂- ;
R représente de l'hydrogène, un alkyle en C₁-C₄ ;
Z¹ et Z² représentent de l'hydrogène, un alkyle en C₁-C₁₂, un groupe polymérisable ou un radical qui porte un groupe polymérisable ;
m à p valent 0 ou 1,
les radicaux A¹ à A⁴, Y¹ à Y⁷, ainsi que Z¹ et Z², pouvant être identiques ou différents et, dans le cas où un ou plusieurs des indices m à p valent zéro, au moins l'un des radicaux Y, respectivement voisins de A, représentant une liaison chimique.

6. Substances de dopage chirales selon la revendication 5 les substituants ayant la signification suivante :
R¹ représente Z¹-Y¹-(A¹)ₘ-Y²-M¹-Y³-(A²)ₙ-Y⁴ ;
R² représente Z²-Y⁵-(A³)ₒ-Y⁶-M²-Y⁷-(A⁴)ₚ-Y¹¹- ;
A¹ et A³ représentent un radical espaceur d'une longueur de chaîne de 1 à 6 atomes de carbone ;
Y¹ à Y⁷ représentent une liaison chimique, -O-, -S-, -C(=O)-, -C(=O)-O-, -O-C(=O)-, -CH=CH-C(=O)-O-, -O-C(=O)-O-, -C(=O)-N(R)- ou -(R)N-C(=O)-, -CH₂-O-, -O-CH₂-, -CH=N-, -N=CH- ou -N=N- ;
Y¹¹ représente une liaison chimique, -C(=O)-, -O-C(=O)-, -CH=CH-C(=O)-, -(R)N-C(=O)-, -CH₂-, -O-CH₂- ;
M¹ et M² représentent un radical mésogène choisi dans le groupe constitué de : 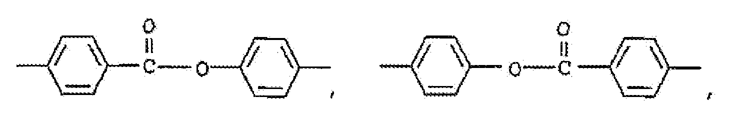 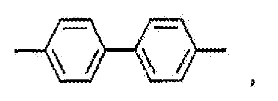 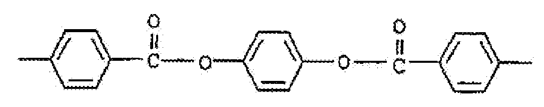 et 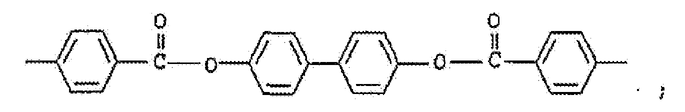
Z¹ et Z² représentent de l'hydrogène, un alkyle en C₄-C₁₀, un groupe polymérisable ou un radical qui porte un groupe polymérisable ;
m vaut 0 ou 1 ;
n vaut 0 ;
o vaut 0 ou 1 ;
p vaut 0,
au moins l'un des radicaux Y³ et Y⁴ ou Y⁷ et Y¹¹ représentant une liaison chimique.

7. Substances de dopage chirales selon l'une quelconque des revendications 5 ou 6 de la formule générale Ia : 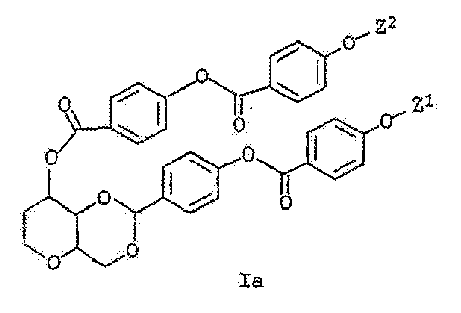 dans laquelle les substituants ont, indépendamment les uns des autres, la signification suivante :
Z¹ et Z² représentent de l'hydrogène, un alkyle en C₄-C₁₀, un groupe polymérisable ou un radical qui porte un groupe polymérisable.

8. Compositions de cristaux liquides cholestériques, comprenant :
a) au moins un monomère de cristal liquide chiral de la formule générale I, selon la revendication 1 : 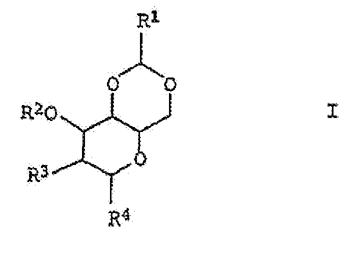 ou
b) un mélange constitué de :
b₁) au moins un monomère polymérisable de cristal liquide achiral de la formule générale II :
Z⁴-Y¹³-(A⁷)ₛ-Y¹⁴-M⁴-Y¹⁵-(A⁸)ₜ-Y¹⁶-Z⁵ II
dans laquelle les variables ont, indépendamment les unes des autres, la signification suivante :
A⁷ et A⁸ représentent un radical espaceur d'une longueur de chaîne de 1 à 30 atomes de carbone ;
M⁴ représente un groupe mésogène ;
Y¹³ à Y¹⁶ représentent une liaison chimique, -O-, -S-, -C(=O)-, -C(=O)-O-, -O-C(=O)-, -CH=CH-C(=O)-O-, -O-C(=O)-O-, -C(=O)-N(R⁷)- ou -(R⁷)N-C(=O)-, -CH₂-O-, -O-CH₂-, -CH=N-, -N=CH- ou -N=N- ;
R⁷ représente de l'hydrogène, un alkyle en C₁-C₄ ;
s vaut 0 ou 1 ;
t vaut 0 ou 1 ;
Z⁴ et Z⁵ représentent de l'hydrogène, un alkyle en C₁-C₁₂, un groupe polymérisable ou un radical qui porte un groupe polymérisable ;
les radicaux A⁷ et A⁸, ainsi que Y¹³ à Y¹⁶, pouvant être identiques ou différents, au moins l'une des variables Z⁴ ou Z⁵ représentant un groupe polymérisable ou in radical qui porte un groupe polymérisable et, dans le cas où l'un des deux indices, voire les deux indices s et t valent zéro, au moins l'un des radicaux Y¹³ et Y¹⁴ ou Y¹⁵ et Y¹⁶ représentant une liaison chimique, et
b₂) au moins un monomère de cristal liquide chiral de la formule générale I, selon la revendication 1.

9. Compositions de cristaux liquides cholestériques selon la revendication 8, comprenant comme monomère de cristal liquide chiral a) ou b₂) au moins un composé chiral de la formule générale Ia : 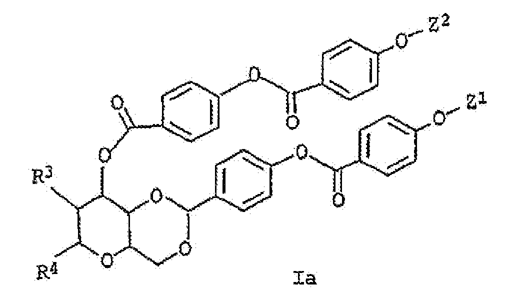 dans laquelle les substituants ont, indépendamment les uns des autres, la signification suivante :
R³ représente de l'hydrogène ;
R⁴ représente de l'hydrogène ;
Z¹ et Z² représentent de l'hydrogène, un alkyle en C₄-C₁₀, un groupe polymérisable ou un radical qui porte un groupe polymérisable.

10. Compositions de cristaux liquides cholestériques selon l'une quelconque des revendications 8 ou 9, **caractérisées en ce qu'**elles réfléchissent la lumière polarisée de façon circulaire vers la gauche dans la plage des U.V.

11. Utilisation de compositions de cristaux liquides cholestériques définies selon l'une quelconque des revendications 8 à 10, comme filtre à U.V. pour la fabrication de préparations cosmétiques et pharmaceutiques destinées à protéger la peau ou les cheveux d'êtres humains des rayons du soleil, seules ou conjointement avec des composés absorbants dans la plage des U.V. et connus en soi pour les préparations cosmétiques et pharmaceutiques.

12. Utilisation de compositions de cristaux liquides cholestériques selon la revendication 11, pour la fabrication de réflecteurs d'U.V. photostables.

13. Utilisation de compositions de cristaux liquides cholestériques définies selon l'une quelconque des revendications 8 à 10, comme stabilisants U.V. pour la fabrication de formulations cosmétiques et pharmaceutiques.

14. Utilisation de compositions de cristaux liquides cholestériques selon l'une quelconque des revendications 11 à 13, sous forme de pigments.

15. Pigments contenant des compositions de cristaux liquides cholestériques définies selon l'une quelconque des revendications 8 à 10.

16. Pigments selon la revendication 15, **caractérisés en ce qu'**il s'agit de pigments multicouches.

17. Préparations cosmétiques et pharmaceutiques pour protéger l'épiderme ou les cheveux d'êtres humains contre la lumière U.V. dans la plage de 280 à 400 nm, **caractérisées en ce qu'**elles contiennent dans un support approprié sur le plan cosmétique ou pharmaceutique, seules ou conjointement avec des composés absorbants dans la plage des U.V. connus en soi pour les préparations cosmétiques et pharmaceutiques, des quantités efficaces de compositions de cristaux liquides cholestériques définies selon l'une quelconque des revendications 8 à 10 comme filtre à U.V. photostable.

18. Préparations cosmétiques et pharmaceutiques selon la revendication 17, contenant des compositions de cristaux liquides cholestériques sous forme de pigments comme filtre à U.V.

19. Préparations cosmétiques et pharmaceutiques selon l'une quelconque des revendications 17 ou 18, contenant des compositions de cristaux liquides cholestériques sous forme de pigments multicouches comme filtre à U.V.
